# EUROPEAN PATENT SPECIFICATION

(11) **EP 1 488 000 B1**
(45) Date of publication and mention of the grant of the patent: **12.03.2014**
(21) Application number: 03716617.0
(22) Date of filing: 14.03.2003
(51) Int. Cl.: C12Q 1/68, G01N 21/64, G01N 33/553

(54) **SIGNAL AMPLIFICATION BY HYBRID CAPTURE**
SIGNALAMPLIFIKATION DURCH HYBRIDEINFANG
AMPLIFICATION DE SIGNAL PAR CAPTURE D'HYBRIDE

(30) Priority: 15.03.2002 US 98851
(43) Date of publication of application: 22.12.2004
(73) Proprietor: QIAGEN Gaithersburg, Inc., Gaithersburg, MD 20878 (US)
(72) Inventor: LAZAR, James, G., Bethesda, MD 20817 (US)
(74) Representative: Schnappauf, Georg
(86) International application number: PCT/US2003/008033
(87) International publication number: WO 2003/078966

(56) References cited:
- EP-A2- 0 516 529
- WO-A-00/18962
- WO-A-95/08000
- WO-A-97/13874
- WO-A-98/18488
- JP-A- 2000 221 194
- US-A- 4 271 140
- US-B1- 6 306 598

## Description

The field of the invention relates to detection assays and signal amplification. The assays allow the detection of minute amounts of a target analyte or biomolecule by amplifying the signal generated from recognition of the presence of the target analyte or biomolecule.

### BACKGROUND OF THE INVENTION

Many different types of assay methods for detecting and/or measuring a target molecule in a sample are known in the art. Detection of a particular target molecule (e.g., an antigen, such as a pathogen or a hormone or a single-stranded nucleic acid target) in a fluid sample may be accomplished using a variety of binding assays, e.g., immunoassays, immunohistochemistry, or DNA hybridization assays. Generally, such an assay involves reaction of the test sample with a specific binding reagent (e.g., a specific antibody) and with a reagent which facilitates the direct or indirect quantitative measurement of the amount of the analyte or target of interest in the test sample. Such methods include immunological basis, i.e., they involve measuring the binding of an antibody or antibody fragment to a complementary ligand, e.g., a drug or other molecule. There are several immunoassays, immunohistochemical assays, and binding assays well known in the art for detecting and/or measuring target molecules, such as antigens, antibodies, haptens, hormones, drugs, toxins, and chemicals. Binding molecules may comprise, for example, a member of a ligand-receptor pair (i.e., a pair of molecules capable of specific binding interactions), antibody-antigen, enzyme-substrate, nucleic acid-nucleic acid, protein-nucleic acid, or other specific binding pairs and members thereof as known in the art. Binding molecules may be designed so that they have enhanced affinity for the target analyte. Binding molecules may be linked or coupled to a detectable label or indicator for detection by means known in the art. Means for coupling labels or indicators to binding molecules are also well known in the art. Radioimmunoassay techniques are numerous and highly sensitive. Additionally, several enzyme-immunoassays are commonly known, including, but not limited to, competitive antibody and sandwich procedures.

Although there are many systems for the quantitative and qualitative analysis of target substances known in the art, there is a need for simpler assays with improved sensitivity. Techniques that allow specific detection of target analytes or biomolecules are important for many areas of research, as well as for clinical diagnostics. Central to most detection techniques are ligands that dictate specific and high affinity binding to a target molecule of interest. In immunodiagnostic assays, antibodies mediate specific and high affinity binding, whereas in assays detecting nucleic acid target sequences, complementary oligonucleotide and nucleic acid probes may be used. To date, antibodies have been able to provide molecular recognition for a wide variety of target molecules and have been the preferred choice of the class of ligands for developing diagnostic assays.

Attempts have been made to increase the sensitivity of nucleic acid assays by target amplification. Methods of amplifying nucleic acid sequences are commercially available. These methods include the polymerase chain reaction (PCR), the ligation amplification reaction (LCR), and the transcription based amplification reaction (TMA). PCR technology is described in PCR Protocols A Guide to Methods and Applications by Michael A. Innis, David H. Gelfand, John J. Sninsky and Thomas J. White, pp. 39-45 and 337-385 (Academic Press, Inc., Harcourt Brace Jovanovich, Publishers, 1990). PCR technology is also described by Marx, J.L., Science 140:1408-1410 (1988) and in U.S. Patent Nos. 4,683,195 and 4,683,202, to Mullis. Ligation amplification reaction is described by Wu, D.Y and Wallace, R.B, Genomics 4:560-569 (1989) and Barringer, K.J., et al., Gene 89:117-122 (1990). Transcription based amplification reaction is described by Kwoh, D.Y., et al., Proc. Natl. Acad. Sci. USA 86:1173-1177 (1989). These methods have the advantages of high sensitivity, but the disadvantages of having a lengthy, tedious, and expensive sample preparation, being prone to false-positive results from reaction product contamination, and having the inability to accurately quantify the initial amount of target nucleic acids. Amplification reaction products are most often detected by a hybridization assay.

Signal amplification assays for nucleic acid detection such as Hybrid Capture (DIGENE, Corp.; Gaithersburg, MD) and branched-DNA (Bayer Corp.; Tarrytown, NY) provide advantages over target amplified nucleic acid methods in that they do not require highly purified samples, and they are not subject to sample inhibition or contamination issues. At best, however, the sensitivity of these methods ranges from a few hundred copies to several thousand copies depending on the length of the target sequence. For both methods, the sensitivity for very short nucleic acid targets is poor. No general target amplification methods are available for proteins or other biomolecules, rather only very limited methods of signal amplification exist such as Enhanced Chemiluminescence (ECL™; Amersham; Piscataway, NJ) and Western-*Star*™ and Westem-*Light*™ Immunodetection systems (TROPIX; Bedford, MA).

JP 2000221194 and WO 0018962 describe signal amplification systems using biotin and streptavidin. The disadvantages of prior techniques for detecting analytes or biomolecules are several fold. Firstly, they require a large quantity of highly purified biomolecules, nucleic acids (i.e. RNA or DNA), proteins, and the like. Purification requires additional steps which are time consuming and labor intensive. Consequently, there is a need for a method of detection for quantitative analysis of biological molecules, including DNA, RNA, protein, and the like, that is accurate, both time and cost efficient, and capable of screening one or more sample molecules with great sensitivity and minimal non-specific binding.

Detecting analytes such as proteins is extremely important in understanding human physiology, development of drugs or medicines, and diseases that occur on a protein level. Now that the human genome has been sequenced, proteomics, or the large-scale study of the proteins that the genes encode, plays an increasingly critical role in health and diseases, where proteins may be used as diagnostic markers, therapeutic agents, and targets for monoclonal antibody and small-molecule drug development.

Therefore, it is an object of the present invention to provide a method of detecting and measuring the amount of one or more molecules or analytes, including, but not limited to RNA, DNA, protein, chemicals, and drugs, that is easy to use, highly specific, accurate, and sensitive for screening molecules or analytes. In order to expedite the screening process, it is a further object of the present invention to provide a method of detecting and measuring molecules or analytes by high throughput and highly parallel screening of analytes or biomolecules, where multiple analytes may be processed simultaneously and in parallel

### SUMMARY OF THE INVENTION

The description is limited by its claims. The present invention provides a highly specific and sensitive assay for detection of many different types of analytes or biomolecules. Since a highly purified sample is not necessary, the invention has a simplified sample preparation process, allowing for an accurate detection and measurement of molecules. The method is not subject to any limitations with regard to sample type, purity, or format. Also, molecules need not be directly labeled for detection and measurement, thereby avoiding any interference attributed to the label or the labeling process. The present invention provides an extremely sensitive method for detecting and measuring analytes or molecules, since a very high labeling density may be achieved by utilizing an entity that binds to nucleic acids or nucleic acid hybrids, such as RNA, DNA, or RNA:DNA, DNA:DNA, or RNA:RNA hybrids. Such discriminating sensitivity thereby reduces the amount of sample required for analysis. The present invention is simple and is the first general method for detecting target analytes, including but not limited to, proteins and other biomolecules that are signal-amplified by a hundred to thousand fold. The present invention may be applied to any immunoassay or nucleic acid hybridization assay in which the target analyte can be preferably immobilized directly or indirectly. The method may be applied to any high throughput, highly parallel array formats including, but not limited to microarrays, nucleic acid arrays (gene chips), protein arrays, blots, and bead-based assays.

The present invention relates to detection of small quaritities of a target analyte by signal amplification. Amplification is achieved through the use of one or more binding partners that increase the number of binding sites for signal moieties. Optionally, the binding partners may be stacked, thereby creating even more binding sites for signal moieties. Multiple binding sites are generated by linking the binding partners with a nucleic acid hybrid, such as RNA:DNA, DNA:DNA, or RNA:RNA hybrids. This hybrid is preferably long and thereby possess many recognition sites for a detectably labeled nucleic acid hybrid antibody (referred to herein as a hybrid antibody). Thus, a combination of the use of binding partners and the many recognition sites generated by the hybrid results in a signal amplification system capable of sensitive target analyte detection.

### BRIEF DESCRIPTION OF THE DRAWINGS

FIGURE 1 shows a general scheme for detection of a target analyte by reacting an antibody which has been conjugated to binding molecule A, which is reacted with its binding partner, A'. Binding partner A' is conjugated to a reagent that is reacted with a second antibody which is detectably labeled.
FIGURE 2 shows a general scheme for detection of a target analyte or biomolecule by microarray.
FIGURE 3 shows a general scheme for detection of a target analyte or biomolecule by hybridization and immunodetection.
FIGURE 4 shows a general scheme for detection of a target nucleic acid by hybridization and immunodetection (Hybrid Capture).
FIGURE 5 shows a general scheme for detection of a target nucleic acid by hybridization and immunodetection (HC3/EAS).
FIGURE 6 shows a comparison of average net signal (signal minus noise) between detection by standard protocol versus amplified protocol.
FIGURE 7 shows a correlation in net signal between the amplified protocol and the standard protocol in microarray detection.
FIGURE 8 shows detection of goat IgG by Hybrid Capture signal amplification with 100 µg/mL biotinylated antibody raised against goat.
FIGURE 9 shows detection of goat IgG by Hybrid Capture signal amplification with 10 µg/mL biotinylated antibody raised against goat.

### DETAILED DESCRIPTION OF THE INVENTION

In accordance with the present invention, there may be employed conventional molecular biology, microbiology, and recombinant DNA techniques within the skill of the art. Such techniques are explained fully in the literature. See, e.g., Sambrook et al., "Molecular Cloning: A Laboratory Manual" (1989); "Current Protocols in Molecular Biology" Volumes I-III [Ausubel, R.M., ed. (1994)]; "Cell Biology: A Laboratory Handbook" Volumes I-III [J.E. Celis, ed. (1994)]; "Current Protocols in Immunology" Volumes I-III [Coligan, J.E., ed (1994)]; "Oligonucleotide Synthesis" (M.J. Gait ed. 1984); "Nucleic Acid Hybridization" [B.D. Hames & S.J. Higgins, eds. (1984)]; "Transcription and Translation" [B.D. Hames & S.J. Higgins eds.(1984)]; "Animal Cell Culture" [R.I. Freshney, ed. (1986)]; "Immobilized Cells and Enzymes" [IRL Press, (1986)]; B. Perbal, "A Practical Guide to Molecular Cloning" (1984).

The invention pertains to methods of screening a sample for the presence or absence of at least one target analyte. In particular, the invention relates to a signal amplification system which combines the use of binding partner pairs and a hybrid antibody to detect low levels of a particular analyte.

The phrase "binding partner pairs" or "binding pair" as used herein, relates to molecules which bind together under preselected binding conditions, wherein a binding molecule A binds to its partner A'.

Specific binding pairs suitable for use in practicing the invention may be of the immune or non-immune type. Immune specific binding pairs are exemplified by antigen/antibody systems or hapten/anti-hapten systems. The antibody member, whether polyclonal, monoclonal, derivative of an antibody, an immunoreactive fragment, or immunofragment thereof, of the binding pair may be produced by customary methods familiar to those skilled in the art.

The terms "immunoreactive antibody fragment", "immunoreactive fragment", and "immunofragment" mean fragments which contain a binding region specific for a target analyte, binding partner, antigen and the like. Such fragments may be Fab-type fragments which are defined as fragments devoid of the Fc portion, for example, Fab, Fab' and F(ab')₂ fragments, or may be so-called "half-molecule" fragments obtained by reductive cleavage of the disulfide bonds connecting the heavy chain components of the intact antibody. Additionally, the Fc portion of IgG: Protein A/ Protein G/ Protein A/G are further examples of binding pairs. If the antigen member of the specific binding pair is not immunogenic, it may be covalently coupled to a carrier protein to render it immunogenic. Further, the phrase "derivative of an antibody" refers to a derivative or protein containing the binding domain of an antibody, where the antibody is immunospecific to double-stranded hybrids, such as but not limited to RNA/DNA; DNA/DNA; or RNA/RNA, and mimics thereof, where "mimics" as defined herein, refers to as molecules that behave similarly to DNA or RNA or RNA/DNA, DNA/DNA, or RNA/RNA hybrids. Other immune binding pairs include, but are not limited to, digoxigenin with anti-digoxigenin; 5-bromodeoxyuridine (BrdU) with anti-BrdU; Dinitrophenyl (DNP) with anti-DNP; Fluorescein isothiocyanate (FITC) with anti-FITC; N-2-Acetylaminofluoren (AAF) with anti-AAF; and N-2-Acetylamino-7-iodofluoren (AAIF) with anti-AAIF. Other such binding pairs are commonly known in the art.

Non-immune binding pairs include systems wherein the two components share a natural affinity for each other but are not antibodies. Exemplary non-immune binding pairs include, but are not limited to: biotin with avidin or biotin with streptavidin; folic acid with folate binding protein; sialic acid, carbohydrates, glycoproteins with lectins (Con A); oligo- or poly-dA with oligo- or poly-dT; oligo- or poly-dC with oligo- or poly-dG; and Phenylboronic acid (PBA) with Salicylhydroxamic acid (SHA). Also included are non-immune binding pairs which form a covalent bond with each other. Exemplary covalent binding pairs include sulfhydryl reactive groups such as maleimides and haloacetyl analogues and amine reactive groups such as isothiocyanates, succinimidyl esters, sulfonyl halides, and coupler dyes such as 3-methyl-2-benzothiazolinone hydrazone (MBTH) and 3-(dimethyl-amino)benzoic acid (DMAB); aldehyde and ketone moities with hydrazides; sulfhydryl moiety with maleimides; and heavy metals (Hg²⁺) with thiols (glutathione); and amino moieties with N-hydroxysuccinimide esters. Other such binding pairs are commonly known in the art.

The disclosed assay of the present invention may be used to detect any target analyte, or combination of target analytes in a sample, wherein the term "target analyte" and "analyte" used interchangeably, as defined herein, refers to, but is not limited to, nucleic acids, amino acids, analogues, proteins, peptides, antibodies, hormones, glycoproteins, proteoglycans, sugars, lipids, sterols, drugs, chemicals, toxins, cells, and the like. Furthermore, analytes include "biological molecules" and "biomolecules", used interchangeably herein. The present method may detect a wide variety of target biomolecules. Specifically, examples of such biomolecules include, but are not limited to, nucleic acids, proteins, peptides, glycoproteins, proteoglycans, saccharides, lipids or complexes thereof. Target biomolecules for detection are selected based on the needs and purpose of the detection. In general, a biomolecule of interest may be chosen based on known criteria for selecting a biomolecule for detection. For example, a particular biomolecule may be associated with a pathogen, a disease state, or a predisposition to a disease, and detection of such a biomolecule, comprising a nucleic acid molecule, for example, may have a diagnostic value. In particular, mRNA specific to tumor cells or normal cells may be detected.

"Nucleic acid" refers to deoxyribonucleotides or ribonucleotides and polymers thereof, from any source, including, but not limited to synthetic or derived from bacteria, yeast, viruses, and the cells or tissues of higher organisms such as plants or animals, and unless otherwise limited, may encompass known analogues of natural nucleotides that may function in a similar manner as naturally occurring nucleotides. Peptide nucleic acids (PNAs) are also encompassed within the scope of the term nucleic acid. The term "target nucleic acid" may refer to the specific subsequence of a larger nucleic acid to which the probe is directed or to the overall sequence (e.g., gene or mRNA) whose detection is desired. The difference in usage will be apparent from the context. For example, the target nucleic acid is a DNA or DNA analogue (mimic) to which a particular RNA can specifically hybridize. The DNA or DNA mimic of the target can be, e.g., a synthetic oligomer, a full-length cDNA, a less-than full length cDNA, or a gene fragment, where a "mimic" as defined herein. However, the target analyte is not limited to DNA or RNA.

A "nucleic acid" is further defined herein as a single- or double-stranded nucleic acid ranging in length from about 2 to about 100, 000 bases. As also used herein, the term "nucleic acid" refers to oligonucleotides, cDNA, mRNA, amplicons, plasmids, and the like. An "oligonucleotide" or "oligo" is one preferred nucleic acid probe comprising at least about 6 to about 60 nucleotides, preferably about 15 to about 30 nucleotides, and more preferably about 20 to about 25 nucleotides, which may be used in PCR amplification or a hybridization assay, or a microarray. As used herein, oligonucleotide is substantially equivalent to the terms "amplimers" and "oligomers", as commonly defined in the art, and may be used as "primers" and "probes" as described herein. Also, unless otherwise limited, the term "nucleic acids" encompasses known analogues of natural nucleotides which have similar functional properties as the reference nucleic acid and may be metabolized in a manner similar to naturally occurring nucleotides. In addition, a particular nucleic acid sequence also implicitly encompasses conservatively modified variants thereof (e.g. degenerate codon substitutions) and complementary sequences as well as the sequence explicitly indicated.

The detection of nucleic acids also includes that of mutations, deletions, insertions of single nucleotide polymorphisms, and other polymorphisms. As mentioned above, the method of the present invention also allows the detection of other biological molecules in a sample. These biomolecules may be generated by molecular biological, biochemical, or chemical methods.

A "sample" or "target sample" as used interchangeably herein, includes biological material and/or synthetic material of molecules, preferably biomolecules. The sample may be derived from sources including, but not limited to patients, tissues, organs, cells, or may be synthesized or amplified by conventional techniques. A sample may comprise chemicals, hormones, drugs, toxins, total genomic DNA, total RNA, genomic DNA or mRNA from, for example chromosomes, or selected sequences (for example, particular promoters, genes, amplification or restriction fragments, cDNA, etc.) within particular amplicons or deletions. Since samples may also be in a crude or unpurified state, the sample preparation or processing is simplified. By using samples found in a more natural state, accurate expression detection is achieved.

Alternatively, a sample for the disclosed method of the invention may be from any source containing or suspected of containing target analytes or biomolecules. The source of a target analyte or biomolecule may be in purified or non-purified form. Preferred types of samples, or sources of samples, suitable for use in the disclosed method are those samples already known or identified as samples suitable for use in other methods of analyte or biomolecule detection. Many such samples are commonly known in the art. For example, the sample may be from an agricultural or food product, or may be a human or veterinary clinical specimen. Samples may be a biological fluid such as plasma, serum, blood, urine, sputum, cell lysate, or the like. The sample may contain bacteria, yeast, viruses and the cells or tissues of higher organisms such as plants or animals, suspected of harboring a target analyte or molecule of interest. Methods for the extraction and/or purification of analytes or biomolecules have been described and are known in the art. For example, nucleic acids have been described by Maniatis et al., Molecular Cloning: A Laboratory Manual (New York, Cold Spring Harbor Laboratory, 1982).

The sample, such as, but not limited to an analyte or biomolecule sample, may be extracted from particular cells or tissues. The tissue sample from which the analyte or biomolecule sample is prepared may be taken from a patient suspected of having the disease associated with the molecules being detected. In some cases, the biological molecules, for example, nucleic acids, may be amplified using standard techniques such.as PCR, prior to the hybridization. The nucleic acid sample may be a tissue extract or cell lysate sample prepared by methods known in the art. The sample is prepared such that biological molecules of interest are released from cells and are available for hybridization.

Target biological molecules of interest for use in the disclosed method may come from various sources, both natural and synthetic. For example, various types of RNA include messenger RNA, ribosomal RNA, nucleolar RNA, transfer RNA, viral RNA and heterogeneous nuclear RNA, total genomic DNA, cDNA, proteins, peptides, or fragments thereof. In addition, whole naturally-occurring entities or fragments thereof may be used.

Sensitivity is a key element in the binding assay of the present invention. In particular, "sensitivity" is defined herein as the minimal detectable dose, namely the smallest mass of analyte or biomolecule, that generates a statistically significant change in the signal generated by the assay when compared to that obtained in the absence of analyte or biomolecule. The increased sensitivity of the present binding assays allows small amounts of analyte to be detected, since in many situations, analytes or biomolecules, such as hormones, drugs, microorganisms, toxins, pollutants, or genetic materials, apply their effects at low concentrations and are difficult to detect.

A "reagent" as the term is used herein is a molecule which has a repeating motif created by the structure or conformation of the molecule. Reagents may be any repeating molecule for which a binding partner is available. The binding partner recognizes the repeating motif. One example of such a reagent is a nucleic acid or nucleic acid hybrid, such as, for example an RNA, DNA, or an RNA:DNA, DNA:DNA, RNA:RNA, or an RNA:DNA vector hybrid such as an M 13 RNA:DNA vector hybrid. The binding protein for this reagent may be antibodies specific for the three dimensional conformation of these nucleic acid structures. Alternatively, other reagents may include molecules such as polymerized proteins, polyamino acids, PEG or triglycerides, and linear or three dimensional polymers, wherein the reagent is reacted with a detectably labeled compound. Other binding proteins that are preferably detectably labeled and specific for reagents such as those described above, include, but are not limited to, antibodies specific for polymerized proteins, polyamino acids, PEG or triglycerides, linear or three dimensional polymers, and repeating polymers. Furthermore, one embodiment of the present invention relates to other binding proteins or entities that bind RNA or DNA, or RNA:DNA, DNA:DNA, or RNA:RNA hybrids. For example, proteins that bind single-stranded or double-stranded nucleic acids, such as, but not limited to DNA single-stranded binding protein. Psoralen, ethidium bromide, propidium iodide, Hoescht dyes, SYBR Green (Molecular Probes; Eugene, OR), cis-platinum derivatives, and the like, bind to nucleic acids and may be detected directly or indirectly.

A wide variety of labels or reporters are available for detecting the biomolecule of interest. Reporters may bind directly or indirectly to the reagent. Reporters can be a radioactive isotope, such as, ¹²⁵I, enzymes, fluorogenic, chemiluminescent, or electrochemical materials. Additionally, micro or nano-transponders and nanocrystals may be used, including, but not limited to, fluorescent and light-scatterers. One example of a fluorescent intercalating direct reporter dye is propidium iodide. Additionally, other markers that can be used to accelerate detection of amplified molecules include chemiluminescent markers, immuno-affinity tags such as c-myc, affinity tags such as cellulose-binding domain, streptavidin tag, biotin/streptavidin or any whole or part marcomolecule with a matching fit, reporter enzymes with chromogenic, luminescent, fluorescent or other tracer capabilities, or adducts that confer special and distinct properties on the target (G. H. Keller, M. M. Manak, DNA Probes, 2nd Edition, Stockton Press, New York, N., pp. 1-659, 1993).

The use and choice of reporter molecules are commonly known in the art. Examples of reporter enzymes which can be used to practice the invention include hydrolases, lyases, oxidoreductases, transferases, isomerases, and ligases. Some preferred examples are phosphatases, esterases, glycosidases, and peroxidases. Specific examples include alkaline phosphatase, lipases, beta-galactosidase, and horseradish peroxidases. Further, examples of fluorescent reporter molecules include, but are not limited to fluorescein isothiocyanate (FITC), rhodopsin, cyanine dyes such as, but not limited to, CY3 and CY5, phycoerythrin (PE), and Alexa dyes (Molecular Probes; Eugene, OR).

Several substrates or reactants are currently available for highly sensitive detection of target analytes or biomolecules. The term "substrate" as used herein refers to any substance or material on which an enzyme acts. Preferably, chemiluminescent substrates are used. An exemplary binding substrate may be a conjugate of biotin coupled to tyramine via a N-hydroxysuccinimido linker molecule. The binding substrate can be synthesized using commonly known methods in the art. (M. N. Bobrow, et. al., J. Immunol. Methods, 125, 279, (1989)).

In particular, 1,2-dioxetane substrates for methods such as discovery research and high-throughput pharmaceutical screening, genomic and proteomic functional assays, reporter-gene assays to monitor gene expression, second-messenger quantitation, protein kinase assays, and protein-protein interaction analysis are available for use. Alkaline phosphatase (AP) enzyme labels may be used with 1,2-dioxetane chemiluminescent substrates for highly sensitive target analyte detection. Specifically, dioxetanes are four-membered cyclic peroxides that have been suggested to be short-lived, unstable intermedicates in oxidation reactions resulting in chemiluminescence (F. McCapra. Quarterly Review of the Chemical Society. 20:485-510, 1966). For example, adamantyl-1,2-dioxetane phosphates are direct substrates for AP. Briefly, hydrolytic dephosphorylation of adamantyl 1,2-dioxetane phosphate substrates by AP results in a light emitting excited-state anion. Examples of 1,2-dioxetane substrates for AP include those commercially available from TROPIX (AMPPD, CSPD, CDP, and CDP-Star); Luminigen, Inc. (Luminigen PPD, Lumi-Phos, Lumi-Phos 530, and Lumi-Phos Plus); and those 1,2-dioxetane substrates for other enzymes, such as but not limited to β-galactosidase and other β-glycosidases. Macromolecular enhancers may additionally be used to significantly improve the intensity of luminescence produced by 1,2-dioxetane substrates.

For purposes of increasing the signal-to-noise ratio of the technique, some embodiments of the invention provide for exposure of the substrate to a labeled binding pair, where for example, an antibody specific for the substrate is conjugated to a binding molecule (A) and reacted with a binding partner (A') that is conjugated to a reagent that is reacted with a detectably labeled protein that is specific for the reagent, where the detectably labeled protein is an antibody. For the detection of target, the labeled protein may bind at multiple sites of the reagent, thereby amplifying the signal, providing several times the signal (e.g., fluorescence) compared to the attachment of a single detectably labeled protein at each binding site. This process may be repeated again with additional proteins or antibodies (e.g., goat-mouse-goat, etc.) for further signal amplification.

Many assays are commonly known in the art for the detection and quantitation of target molecules. In particular, immunoassays and immunohistochemistry assays that allow for such detection, include, but are not limited to, radioimmunoassays (RIA), ELISA, indirect immunofluorescence assays, Western blot assays, immunohistochemical assays, chemiluminescent assays, immunoprecipitation assays, dot blot assays, slot blot assays and the like. Also included are assays for detecting nucleic acids, such as but not limited to "Hybrid Capture" (DIGENE, Corp.), which is defined herein as a solution hybridization assay with immunodetection fornucleic acids, and a sandwich hybridization assay for nucleic acid detection, which is defined herein as "HC3/EAS" (DIGENE, Corp.). Labeled antibodies are often used but may be unlabeled depending on the type of assay used. Labels which may be coupled to the antibodies include those known in the art and include, but are not limited to, enzymes (e.g., horseradish peroxidase or glucose oxidase), radioisotopes, fluorogenic substrate (e.g., fluorescein isothiocyanate (FITC), fluorescein isocyanate (FIC), 5-dimethylamine-1-napthalenesulfonyl chloride (DANSC), tetramethyl-rhodamine isothiocyanate (TRITC), lissamine, and the like), chromogenic substrates, cofactors, biotin/avidin, chemiluminescent compounds, phosphorescent compounds, colloidal gold, colored particles and magnetic particles.

As is appreciated by the skilled practitioner, in such cases in which the principal indicating group is an enzyme, additional reagents (e.g., substrates) are required for the production of a detectable signal. Radioactive elements of various classes, such as ¹²⁴I, ¹²⁵I, ¹³¹I, ⁵¹Cr, (gamma ray emitters); ³²P, ³H, ³⁵S (beta emitters), and ¹¹C, ¹⁴C, ¹⁵O, or ¹³N (positron emitters), may also be used as detectable labels. The labeled complex may be detected visually, with a spectrophotometer, or by another detector, depending on the labeling or indicating group. To facilitate detection of resulting binding of the antibody, or the other entity specific for double-stranded hybrids, to the hybrid, the antibody will normally be labeled with a detectable chemical group. Examples of detectable chemical groups that may serve as labels are enzymatically active groups, such as coenzymes, enzyme substrates, enzyme inhibitors, and enzymes themselves, fluorescers, chromophores, luminescers, specifically bindable ligands such as biotin or haptens which are detectable by binding of labeled avidin or labeled hapten antibodies, and radioisotopes.

In one embodiment of the present invention, high throughput or highly parallel screening of analytes using a "solid matrix" or "solid phase" to detect and measure multiple analytes simultaneously is preferred. A "solid matrix" or "solid phase" relates to any surface to which analytes, biomolecules, antibodies, or reagents may be bound and subsequently detected either directly or indirectly. Solid matrices, phases, or supports may have any useful form including thin films or membranes, beads, bottles, microwell or microtiter plates, dishes, slides, blotting material, filters, fibers, woven fibers, shaped polymers, particles, microparticles, dip-sticks, test tubes, chips, microchips, and even a liposome. Preferred substrate forms for a solid phase are microtiter plates, silicon chips, glass slides, beads, and microparticles. In particular, these solids include those made of plastics, resins, polysaccharides, silica or silica-based materials, functionalized glass, modified silicon, carbon, metals, inorganic glasses, membranes, nylon, natural fibers such as silk, wool and cotton, and polymers. Solid phases or solid supports may be porous or non-porous. Additionally, bead based arrays may be used, such as, but not limited to those provided by Luminex (Austin, TX), Pharmaseq (Monmouth Junction, NJ), and Illlumina (San Diego, CA). In some embodiments, the material comprising the solid support has reactive groups such as carboxy, amino, hydroxy, aldehyde, etc., which are used for covalent or non-covalent attachment of the probes. Suitable polymers may include, but are not limited to, polystyrene, polyethylene glycol tetraphthalate, polyvinyl acetate, polyvinyl chloride, polyvinyl pyrrolidone, polyacrylonitrile, polymethyl methacrylate, polytetrafluoroethylene, butyl rubber, styrenebutadiene rubber, natural rubber, polyethylene, polypropylene, (poly)tetrafluoroethylene, (poly)vinylidenefluoride, polycarbonate and polymethylpentene. Preferred polymers include those outlined in U.S. Pat. No. 5,427,779 to Elsner et al., hereby expressly incorporated by reference. Solid phases and solid supports include, and are not limited to, any solid material to which the probes, primers, oligonucleotides, proteins, peptides, or the like, may be coupled or adhered.

"Microarrays" refer to an orderly arrangement of distinct molecules or substances including, but not limited to, biological molecules such as RNA, DNA, protein, or the like, or chemicals, arrayed or immobilized to a solid phase or support. These microarrays of binding agents, such as but not limited to, oligonucleotides, probes, receptors, antibodies, or any entity reactive with targets, have become an increasingly important tool in the biotechnology industry and related fields. Microarrays comprising a plurality of binding agents or elements are immobilized onto the surface of a solid support in an orderly fashion or pattern, find use in a variety of applications, including drug screening, nucleic acid sequencing, mutation analysis, genomic and proteomic applications, and the like. Elements as used herein in a microarray context, refer to hybridizable nucleic acid sequences, oligonucleotides, primers, probes, and/ or amino acid sequences arranged in a distinct and identifiable manner on the surface of a solid phase or support. Detection of biological molecules through the use of microarrays, for example, is beneficial for analyzing numerous samples and biological molecules, reducing the amount of sample required for analysis, decreasing experimental variability, decreasing sample preparation time, confirming results, and for decreasing costs of such analysis.

A plurality of different molecules or substances may make up microarrays, including but not limited to, biological molecules, such as, cDNA, amplicons, plasmids, proteins, peptides, and the like, as well as cells, drugs, hormones, toxins, and chemicals, wherein plurality encompasses at least two different molecules, wherein the molecules are immobilized to a solid phase in an ordered matrix or structure. In theory, there need be only one component, but in a preferred embodiment there will be at least about 10, more usually at least about 20, frequently at least about 50, desirably about 100 or more, and even about 1,000 or more, but usually not more than about 10⁶, more usually not more than about 100,000, with from about 10 to about 10,000 immobilized to a solid phase or solid support being preferred. While theoretically the number of different components may exceed about 10⁶, due to the ability to specifically have a small amount or volume at a specified finite site, for the most part there is no need to exceed about 10⁶ and such large numbers of different components do add some complexity to the preparation of the microarray. As the number of components immobilized to a solid phase will usually not exceed about 10⁶, the number of individual addressable sites may be substantially larger, depending on the nature of the bound component, the source of the signal, the nature of the signal which is detected, the sensitivity with which the signal may be detected, the nature of the bound microarray, such as the size of the microarray, the manner in which the microarray is produced, and the like. Therefore, microarrays are preferably used for "massive parallel screening", described herein as the simultaneous screening of at least about 10, preferably about 1,000, and more preferably about 10,000, different biological molecule interactions or hybridizations.

Generally, one embodiment of the invention provides a method of signal amplification for detecting the presence or absence of a target analyte in a sample comprising the steps of: reacting a target analyte with a conjugated binding agent or antibody, which in turn, has been conjugated to a binding molecule, A, and the conjugated antibody is reactive with the target analyte, forming a binding agent complex or an antibody complex; and reacting the binding agent complex or antibody complex via the binding molecule, A, with a conjugated binding partner, A', forming a binding pair, where the binding partner is conjugated to a reagent. The binding pair may then be reacted with a reagent-specific binding entity which is detectably labeled, forming a detectable complex to the binding pair, where the binding entity is, for example, an RNA:DNA antibody. Target analytes may comprise of, but are not limited to, a protein, peptide, or mimic thereof, PNA, amino acids, nucleic acids, analogues, glycoproteins, proteoglycans, sugars, lipids, sterols, hormones, drugs, chemicals, toxins, and cells. The binding agent comprises, but is not limited to, oligonucleotides, probes, receptors, antibodies, or any entity reactive with target analytes.

In yet another embodiment of the present invention, a method of signal amplification for the detection of a target analyte comprising the steps of: reacting a target analyte with a conjugated binding agent, forming a binding agent complex, and the binding agent is conjugated to a reagent. The binding agent complex may then be reacted with a reagent-specific binding entity which is detectably labeled, forming a detectable complex to the binding agent complex.

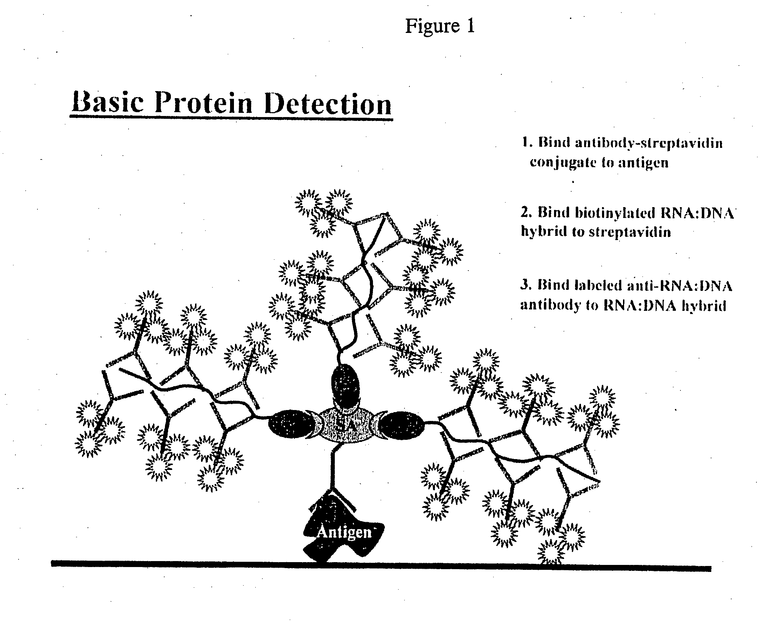

The components of this embodiment, and those embodiments described herein, may be simultaneously or sequentially reacted with each other as described. For example, the binding molecule, A, may be reacted with the binding partner, A', forming a binding pair, prior to the binding molecule reacting with the target analyte. One example of this embodiment relates to the use of streptavidin:biotin binding pairs. Here, detection of an unknown target analyte, preferably, a protein or antigen, for example, is accomplished by reacting the antigen with an antigen-specific antibody conjugated to streptavidin. A biotin-labeled reagent may then be reacted with the complex, forming a streptavidin:biotin complex, or binding pair. One preferred reagent useful in this embodiment, is a RNA:DNA hybrid, where the length of the RNA:DNA hybrid may vary, but is preferably of such a length as to induce, either directly or indirectly, a significant and amplified signal. However, other single- or double- stranded nucleic acid hybrids, such as DNA:DNA or an RNA:DNA vector hybrid, suh as M13, may also be used as reagents. The streptavidin:biotin complex, where the biotin is labeled with the RNA:DNA hybrid reagent, is detected, for example, by the addition of a RNA:DNA hybrid antibody which has been detectably labeled (see FIG. 1). The nucleic acid hybrid antibody, for example RNA:DNA hybrid antibody, is immunospecific to RNA:DNA hybrids, under conditions such that immunospecific binding occurs between the antibody and any RNA:DNA hybrids.

Detection or quantitation of the detectably labeled nucleic-acid hybrid antibody to the corresponding nucleic-acid hybrid is determined by the presence or amount of antibody and thereby indicating the quantity of target analyte present. The detectable label attached to the antibodies raised against the single- or double- stranded hybrid, for example, RNA, DNA, RNA:DNA, or DNA:DNA, may be any chemical, immunological, enzymatic, fluorescent, or chemiluminescent label and may be used for direct or indirect labeling. The nucleic acid hybrid may be pre-made or made by first adding labeled RNA or DNA to the complex and then adding the complementary strand. For example, a biotinylated poly-dT may be first bound to the streptavidin-antibody conjugate followed by poly-A to form the nucleic acid hybrid. The labeled nucleic acid hybrid antibody, as well as streptavidin-antibody conjugate, for example, may be easily prepared by methods commonly known in the art.

This embodiment is readily adaptable for use in a microarray, wherein the analyte to be detected may be any target analyte, and is most preferably a peptide, a protein, a nucleic acid, or mimic thereof. If the analyte is a protein, peptide, or variant thereof, the assay is carried out as described above (Fig. 1), where a either a single analyte or plurality of analytes is detected. If the analyte is a nucleic acid, analogue, or mimic thereof, such as an oligonucleotide, the analyte, or plurality of analytes, can be detected by a microarray assay as well.

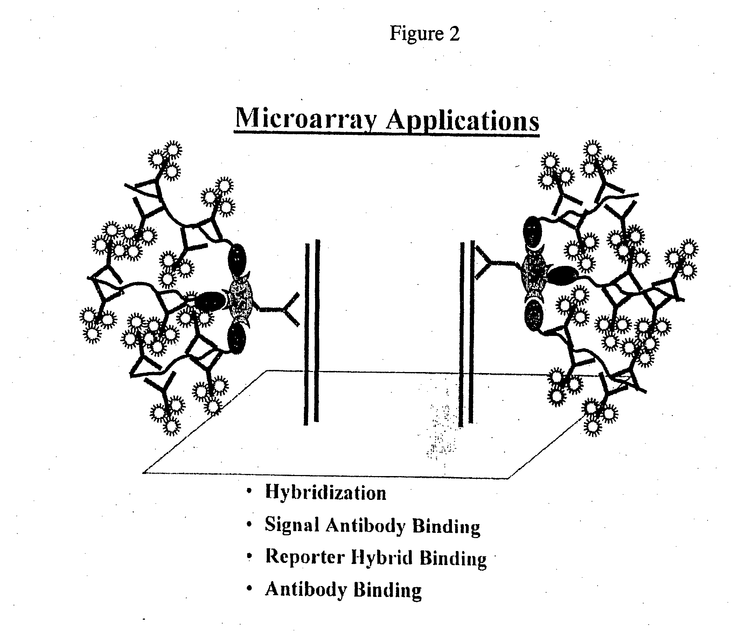

In particular, a method of signal amplification for detection of a target nucleic acid comprising the steps of reacting a target nucleic acid, or plurality of target nucleic acids, with a conjugated antibody, where the antibody is conjugated to a binding molecule, and the antibody is reactive with the target nucleic acid, forming an antibody complex; and reacting the antibody complex with a conjugated binding partner, forming a binding pair, where the binding partner is conjugated to a reagent. A detectably-labeled reagent-specific binding protein is reactive with the binding pair to form a detectable complex, thereby providing signal amplification. In one such embodiment, the target nucleic acid is DNA, and the complement is preferably RNA, and the target nucleic acid is preferably immobilized to a solid phase or support. Alternatively, in this embodiment the target may be RNA and its complement may be DNA. In this embodiment, a RNA:DNA hybrid antibody conjugated to a binding molecule A is reacted with the double-stranded hybrid to form an antibody complex. Alternatively, a double-stranded DNA or RNA may be formed and reacted with an appropriate antibody conjugated to binding molecule A to form a complex. This antibody complex is then reacted with a reagent conjugated to binding partner, A', such that A and A' form a binding pair. A detectably labeled reagent-specific binding protein, preferably in this example an RNA:DNA antibody, is reacted with the reagent such that a detectable complex is formed between the detectably labeled antibody and the reagent. In this embodiment, any binding partner may be employed. Similarly, any detectable label or reporter may be used (see FIG. 2). The components of this embodiment may be simultaneously or sequentially reacted with each other as described. For example, the binding molecule, A, may be reacted with the binding partner, A', forming a binding pair, prior to the binding molecule reacting with the target nucleic acid.

In a further embodiment, a method of signal amplification for detection of a target protein comprising the steps of reacting a target protein, or plurality of target proteins, with a conjugated antibody, where the antibody is conjugated to a binding molecule, and the antibody is reactive with the target protein, forming an antibody complex; and reacting the antibody complex with a conjugated binding partner, forming a binding pair, where the binding partner is conjugated to a reagent. A detectably-labeled reagent-specific binding protein is reactive with the binding pair to form a detectable complex, thereby providing signal amplification. In one such embodiment, the target protein is a peptide, and the target protein is preferably immobilized to a solid phase or support. In this embodiment, a RNA:DNA hybrid antibody conjugated to a binding molecule, A, is reacted with the double-stranded hybrid to form an antibody complex. Alternatively, a double-stranded DNA or RNA may be formed and reacted with an appropriate antibody conjugated to binding molecule A to form a complex. This antibody complex is then reacted with a reagent conjugated to binding partner, A', such that A and A' form a binding pair. A detectably labeled reagent-specific binding protein, preferably in this example an RNA:DNA antibody, is reacted with the reagent such that a detectable complex is formed between the detectably labeled antibody and the reagent. In this embodiment, any binding partner may be employed. Similarly, any detectable label or reporter may be used. The components of this embodiment may be simultaneously or sequentially reacted with each other as described. For example, the binding molecule, A, may be reacted with the binding partner, A', forming a binding pair, prior to the binding molecule reacting with the target analyte. This embodiment is particularly useful for proteomics, where high throughput screening and highly parallel detection of proteins, expedites the screening process for diagnostic and therapeutic uses. For example, identifying proteins that act as diagnostic markers enable physicians to diagnose disease and monitor the progress of treatment.

As discussed above, there are several types of detectable labels any of which may be employed. One preferred embodiment uses luminescent compounds, such as fluorescent compounds and chemiluminescent compounds, because of their ability to emit light. Examples of fluorescent compounds include, but are not limited to, fluorescein and analogues, phycoerythrin, allo-phycocyanin, phycocyanin, rhodamine, Texas Red or other proprietary fluorogens. The fluorogens are generally attached by chemical modification according to methods commonly known in the art. Accordingly, luminescers may be utilized as labels in assays of the present invention. For example, a member of a specific binding pair is conjugated to a luminescer and various protocols are employed. The luminescer conjugate can be partitioned between a solid phase and a liquid phase in relation to the amount of analyte in a sample suspected of containing the analyte. By measuring the luminescence of either of the phases, one can relate the level of luminescence observed to a concentration of the analyte in the sample.

In a further embodiment of the invention, it may be desirable to first capture the analyte onto a solid matrix, directly through an antibody, or through a binding partner pair complex conjugated to a binding molecule or key physical means, such as filtering, drying onto a glass slide, etc. For direct conjugation, methods are known in the art for such binding (Butler, et al. J. Immunol. Meth. 150:77-90, 1992). One form of a solid matrix includes, but is not limited to a microarray that comprises a spotted array onto which analytes or biomolecules may be deposited or synthesized as an array of small dots or elements, as commonly known in the art.

Samples, such as, but not limited to biomolecules, chemicals, drugs, deposited, spotted, or synthesized on a solid phase, are referred to herein as "elements". Typically, an element is less than about 1 mm in diameter. Generally, element sizes are from 1 µm to about 5 mm, preferably between about 1 µm and about 1 mm. Nucleic acid primers for use in the disclosed method may be synthesized using established oligonucleotide synthesis methods. Such methods range from standard enzymatic digestion followed by nucleotide fragment isolation (see for example, Sambrook et al., Molecular Cloning: A Laboratory Manual, 2nd Edition (Cold Spring Harbor Laboratory Press, Cold Spring Harbor, N.Y., 1989) Chapters 5, 6) to purely synthetic methods, for example, by the cyanoethyl phosphoramidite method using a Milligen or Beckman System 1 Plus DNA synthesizer (for example, Model 8700 automated synthesizer of Milligen-Biosearch; Burlington, MA or ABI Model 380B). Synthetic methods useful for making oligonucleotides are also described by Ikuta et al. (Ann. Rev. Biochem. 53:323-356 (1984), (phosphotriester and phosphite-triester methods)), and Narang et al. (Methods Enzymol., 65:610-620 (1980), (phosphotriester method)). Alternatively, target nucleic acids for microarray analysis may be synthesized directly on the microarray by methods known in the art; described for example in U.S. Patent 6,261,776 and WO 99/41007. Two major methods have been disclosed: synthesis using photolabile group protected monomers (Pirrung et al., U.S. Pat. No. 5,143,854 (1992); Fodor et al., U.S. Pat. No. 5,424,186 (1995)) and synthesis using chemical amplification chemistry (Beecher et al., PCT Publication No. WO 98/20967 (1997)). Both methods involve repetitive steps of deprotection, monomer coupling, oxidation, and capping. Other similar methods are known in the art.

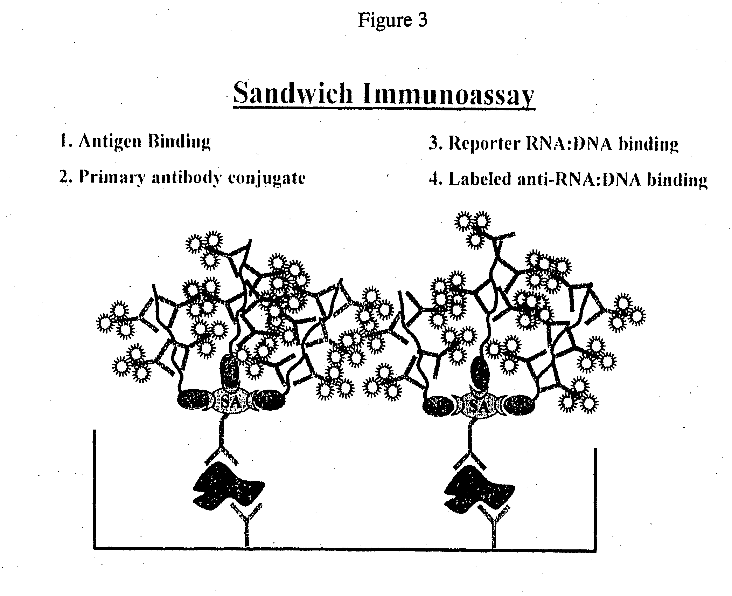

Alternatively, in another embodiment of the invention, a method of signal amplification for detection of the presence or absence of a target analyte comprise antibodies that recognize a target analyte immobilized onto the solid phase. In this embodiment, the immobilized target analyte is reacted with a conjugated antibody, where the conjugated antibody is conjugated to a binding molecule, A, and the conjugated antibody is reactive with the immobilized analyte, thereby forming an immobilized analyte-antibody complex or "sandwich". Further, the immobilized analyte-antibody complex is reactive with a conjugated binding partner, A', forming a binding pair, A:A', where the binding partner is conjugated to a reagent. A detectably-labeled reagent specific binding protein is reactive with the binding pair to form a detectable complex, where detection of the complex provides signal amplification. This particular embodiment may be referred to as a "sandwich" assay, since the analyte is reactive with both an immobilized antibody and a conjugated antibody (see FIG. 3). The components of this embodiment may be simultaneously or sequentially reacted with each other as described. For example, the binding molecule, A, may be reacted with the binding partner, A', forming a binding pair, prior to the binding molecule reacting with the target analyte.

It will be understood by those skilled in the art that the immobilized antibody can be bound directly to the solid phase or indirectly by use of a primary binding antibody or protein, such as streptavidin or protein G, thai is bound to the solid phase and which subsequently binds the anti-hybrid antibody, a derived anti-hybrid antibody, a functional fragment of the anti hybrid antibody, or a derived functional fragment of the anti-hybrid antibody.

Any anti-hybrid antibodies that are specific for a double-stranded hybrid may be used to detect the hybrid complex. It will be understood by those skilled in the art that either polyclonal or monoclonal anti-hybrid antibodies can be used and/ or immobilized on a solid support or phase in the present assay as described below. Monoclonal antibody prepared using standard techniques can be used in place of the polyclonal antibodies. Also included are immunofragments or derivatives of antibodies specific for double-stranded hybrids, where such fragments or derivatives contain binding regions of the antibody.

In a preferred embodiment of the present assay, a polyclonal RNA:DNA hybrid antibody derived from goats immunized with an RNA:DNA hybrid is used. Hybrid-specific antibody is purified from the goat serum by affinity purification against RNA:DNA hybrid immobilized on a solid support. The preferred antibody for detection of RNA:DNA hybrid complexes is preferably prepared by the method of Kitawaga, Y. and Stollar, B.D., Mol. Immunology 19:413-420 (1982) or according to the method set forth in U.S. Patent No. 4,732,847 to Stuart et al., both of which are incorporated herein by reference.

Other suitable methods of producing or isolating antibodies which bind binding sites of the invention, including human or artificial antibodies, can be used, including, for example, methods which select recombinant antibody (e.g., single chain Fv or Fab, or other fragments thereof) from a library, or which rely upon immunization of transgenic animals (e.g., mice) capable of producing a repertoire of human antibodies (see e.g., Jakobovits et al., Proc. Natl. Acad. Sci. USA, 90: 2551-2555 (1993); Jakobovits et al., Nature, 362: 255-258 (1993); Lonberg et al., U.S. Pat. Nos. 5,545,806, Surani et al., 5,545,807).

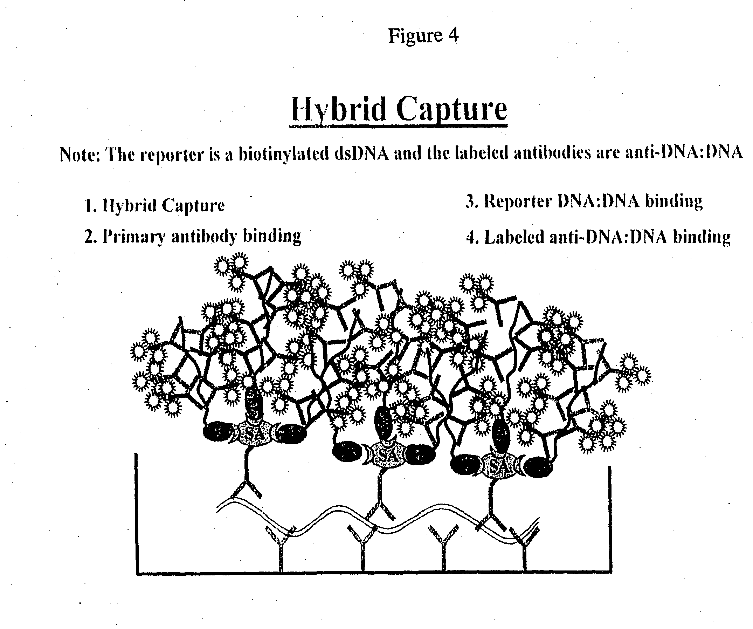

In yet another embodiment, a method of signal amplification for detecting a target analyte, preferably a nucleic acid, comprises an assay that relies on binding the nucleic acid to an immobilized antibody reactive with the target nucleic acid is described. For example, the target nucleic acid is reactive with an immobilized antibody, forming an immobilized nucleic acid. The immobilized nucleic acid is reactive with a conjugated antibody, where the antibody is conjugated to a binding molecule, A, and the antibody is reactive with the target nucleic acid, thereby forming an immobilized nucleic acid-antibody complex. The immobilized nucleic acid-antibody complex is reactive with a conjugated binding partner, A', forming a binding pair, where the binding partner is conjugated to a reagent. A detectably-labeled reagent-specific binding protein is added to the binding pair to form a detectable complex, where detection of the complex provides signal amplification. The target nucleic acid may be DNA, and the complement may be RNA or DNA. If the target is a RNA, the complement may be DNA or RNA. In this way, a binding molecule (A) conjugated for hybrids, preferably RNA:DNA, is added to form an immobilized nucleic acid-antibody complex. A binding partner, A', conjugated to a reagent is reacted with the immobilized nucleic acid-antibody complex to form the A:A' complex. A detectably labeled binding protein reactive with the reagent is added to the A:A' complex for detection (see FIG. 4). The components of this embodiment may be simultaneously or sequentially reacted with each other as described. For example, the binding molecule, A, may be reacted with the binding partner, A', forming a binding pair, prior to the binding molecule reacting with the target nucleic acid or analyte.

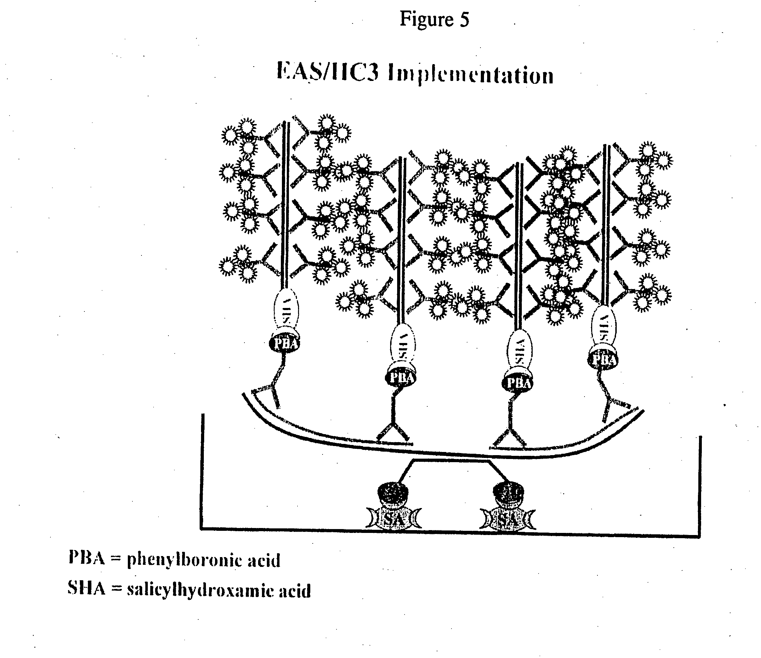

In yet a further embodiment of the invention, it may be desirable to use one or multiple binding pairs in an assay. For example, in an assay to detect a target nucleic acid, a preferred step may be to immobilize a binding molecule, A, to a solid phase or support. Binding partner, A', conjugated to an oligonucleotide complementary to a first portion of a target nucleic acid, is reactive with the immobilized binding molecule, A, forming an A:A' binding pair complex. A portion of the target nucleic acid is hybridized to the oligonucleotide. A nucleic acid probe is hybridized to a second portion of the target nucleic acid, forming a double-stranded hybrid between the probe and target nucleic acid. The double-stranded hybrid is reactive with a conjugated antibody, where the antibody is conjugated to a second binding molecule, B, and the antibody is reactive with the double-stranded hybrid. The antibody may be reactive with either the double-stranded hybrid comprising the target nucleic acid and probe, or the target nucleic acid and oligonucleotide that is conjugated to at least one binding partner, A'. The second binding molecule, is reactive with a conjugated second binding partner, B', forming a second binding pair, B:B', where the second binding partner is conjugated to a reagent. A detectably-labeled reagent-specific binding protein is added to the second binding pair to form a detectable complex, where detection of the complex provides signal amplification. Preferably the reagent is a nucleic acid hybrid and the detectably labeled binding protein is an antibody specific for nucleic acid hybrids. Any number of B:B' complexes may bind to the double-stranded probe:target nucleic acid complex, thereby achieving amplified detection of the target analyte (FIG. 5). If the target nucleic acid is a DNA, then the probe may be RNA or DNA and if the target nucleic acid is a RNA, then the probe may be RNA or DNA. Further, the oligonucleotide may be either a DNA or RNA. Additionally, the components of this embodiment may be simultaneously or sequentially reacted with each other as described. For example, the binding molecule, B, may be reacted simultaneously or sequentially with the binding partner, B', forming a binding pair, prior to or simultaneously with the oligonucleotide conjugated to binding partner, A', simultaneously or sequentially reacting with the target analyte.

Specifically, the multiple binding pair assay, comprising multiple binding pairs or a plurality of binding pairs, having binding molecules (A) and binding partners (A'), utilizes. These binding pairs comprise of conjugated binding partners conjugated to an oligonucleotide, forming bridges between two or more binding partners, where the binding partners are reactive with their respective binding molecules, which are preferably immobilized. In one example, the oligonucleotide may be biotinylated at multiple locations, internally and at the ends, and thereby bind to multiple binding molecules, such as in this example, streptavidin. Further, a plurality or multiple binding pairs may be used for more efficient target nucleic acid capture.

The embodiment of the invention where a target analyte is coupled to a binding molecule, A, and immobilized on a solid matrix may be useful in the detection of labeled analytes present in quantities too small to detect without some amplification. In particular, this embodiment may be useful for microarrays or *in situ* analysis. For example, binding partner, A', conjugated to a reagent-specific antibody is reacted with the analyte, forming an A:A' complex. The reagent is added and a reagent complex with the antibody is formed. This complex is then detected with a detectable reagent-specific binding protein.

Methods of the present invention may be used in the detection, screening, and diagnosis of various diseases, disorders, or conditions in biological samples. Biological samples appropriate for the detection assays of the invention include, but are not limited to, whole blood, serum, plasma and saliva. The methods of the invention may also be used for monitoring the progression of disease or treatment.

All books, articles, and patents referenced herein are incorporated by reference *in toto.* The following examples illustrate various aspects of the invention and in no way intended to limit the scope thereof.

### EXAMPLES

Unless defined otherwise, all technical and scientific terms used herein have the same meanings as commonly understood by one of skill in the art to which the disclosed invention belongs. Although any methods and materials similar or equivalent to those described herein may be used in the practice or testing of the present invention, the preferred methods, devices, and materials are as described. Publications cited herein and the material for which they are cited are specifically incorporated by reference. Nothing herein is to be construed as an admission that the invention is not entitled to antedate such disclosure by virtue of prior invention.

Those skilled in the art will recognize, or be able to ascertain using no more than routine experimentation, many equivalents to the specific embodiments of the invention described herein. Such equivalents are intended to be encompassed by the following claims.

### EXAMPLE 1

### FEASIBILITY OF HYBRID CAPTURE SIGNAL AMPLIFICATION FOR DETECTION OF HYBRIDIZATION ON MICROARRAYS

CaSki cells are a cervical cancer immortal epithelial cell line containing about 600 copies of HPV 16 integrated into the genome. CaSki cells were grown by standard cell culture techniques. Total RNA was extracted and purified from CaSki cells using RNeasy columns (Qiagen).

DNA was amplified from 14 human genes, 4 HPV genes, 3 E. coli genes, 3 B. subtilis genes and 6 Arabidopsis genes using gene-specific primers modified with an amino group on the 5' end. PCR products were purified using Centricon YM100 Columns and were spotted in replicates of 4 onto 3D-Link activated slides (Motorola) using a Genetic Microsystems 418 spotter. After spotting, the slides were dried, boiled in de-ionized water for 5 minutes, dried, and stored.

Anti-RNA:DNA antibody (DIGENE, Corp.) was labeled with biotin according to standard techniques. The antibody was reduced with TCEP (Tris(2-carboxyethyl) phosphine) and reacted with EZ-Link™ PEO-Maleimide Activated Biotin (Pierce). The biotinylated antibody was purified by size exclusion chromatography.

Hybridization solution was prepared by adding 10 µg of CaSki total RNA to 25 µl of HC ExpressArray Hybridization Buffer (DIGENE, Corp.) and brought to a final volume of 50 µl with nuclease-free water. Biotin-labeled anti-RNA:DNA antibody was diluted in HC ExpressArray Antibody Dilution Buffer (DIGENE, Corp.) at a concentration of 100 µg/mL. Streptavidin (Prozyme) was diluted in PBST (Phosphate buffered saline containing 0.05% Tween 20) at a concentration of 5 µg /mL. Biotin-labeled RNA:DNA hybrids (c-myc) were diluted to a concentration of 54.4 µg /mL in 25 uL HC ExpressArray Hybridization Buffer and brought to a final volume of 50 µl using water. Unlabeled anti-RNA:DNA antibody was diluted in HC ExpressArray Antibody Dilution Buffer at a concentration of 100 µg/mL. Cy3-labeled anti-mouse antibody (Zymed) was diluted into PBST at a concentration of 40 µg/mL.

Hybridization was performed by placing a LifterSlip (Erie Scientific) on the spotted microarray, and approximately 50 µl of the Hybridization Solution was pipetted under the LifterSlip. The array was incubated for 3 hours at 75°C in a water bath.

For standard non-amplified detection, the array was removed from the 75°C water bath. The LifterSlip was removed and the array was washed 1 time with PBST. Another LifterSlip was placed on the slide and 45 µl of unlabeled anti-RNA:DNA antibody was pipetted under the Lifter Slip. The array was incubated for 60 minutes at 37°C. The Lifter Slip was removed and the array was washed 1 time with PBST. Another LifterSlip was placed on the slide and 50 µl of Cy3-anti-mouse antibody staining solution was pipetted under the Lifter Slip. The array was incubated for 60 minutes at 37°C. The array was washed 1 time with Wash Buffer (40 mM Tris, 100 mM NaCl, 0.05% Sodium Azide). The array was washed 1 time with HC EAS Enhance Buffer (DIGENE, Corp.) at 55°C. The array was washed 1 time with Wash Buffer. The array was air dried for ∼ 5 minutes. The array was scanned in a GMS 418 Array Scanner at 532 nm.

For signal- amplified detection, the array was removed from the 75°C water bath. The Lifter Slip was removed and the array was washed 1 time with PBST. Another LifterSlip was placed on the slide, and 50 µl of biotin-labeled anti-RNA:DNA antibody was pipetted under the Lifter Slip. The array was incubated for 30 minutes at room temperature. The LifterSlip was removed and the array was washed 1 time with PBST. Another LifterSlip was placed on the slide and 50 µl of streptavidin solution was pipetted under the LifterSlip. The array was incubated for 30 minutes at room temperature. The LifterSlip was removed and the array was washed 1 time with PBST. Another LifterSlip was placed on the slide and 50 µl of biotin-labeled c-myc RNA:DNA hybrids was pipetted under the Lifter Slip. The array was incubated for 30 minutes at room temperature. The LifterSlip was removed and the array was washed 1 time with PBST. Another LifterSlip was placed on the slide, and 50 µl of unlabeled anti-RNA:DNA antibody was pipetted under the LifterSlip. The array was incubated for 30 minutes at room temperature. The LifterSlip was removed and the array was washed 1 time with PBST. Another LifterSlip was placed on the slide and 50 µl of Cy3-anti-mouse antibody staining solution was pipetted under the LifterSlip. The array was incubated for 30 minutes at 37°C. The array was washed 1 time with Wash Buffer. The array washed 1 time with HC Enhance Buffer at 55°C. The array was washed 1 time with Wash Buffer. The array was air dried for - 5 minutes. The array was scanned in a GMS 418 Array Scanner at 532 nm.

The Array Images were analyzed with GenePix Pro 3.0 (Axon Instruments). Median signals and median background (noise) for each spot on the array were calculated. The results from the 4 replicate spots were averaged to generate a mean signal and mean background for each gene on the array. The net signal was calculated by subtracting the mean noise from the mean signal.

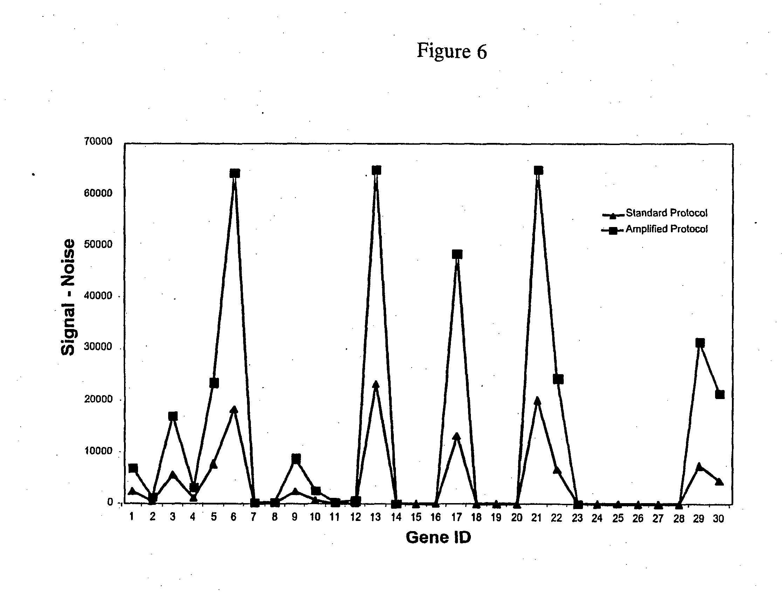

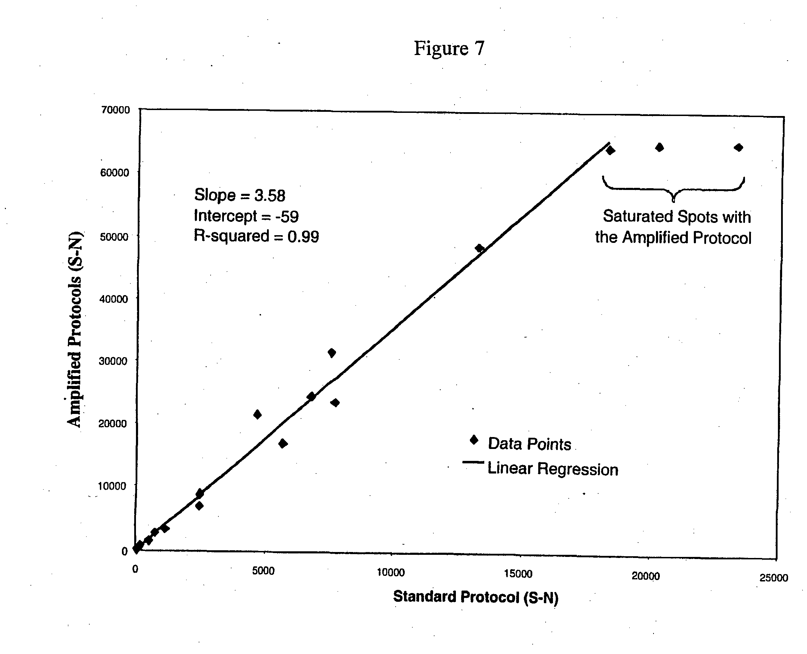

Results are shown in Table 1 and Figure 6. Net signal (signal minus noise) from the amplified protocol increased by 3.35 fold on average compared to the standard protocol for human genes. The amplified protocol did not increase the net signal for non-human genes. Figure 7 shows the correlation in net signal between the amplified protocol and the standard protocol for microarray detection.

**TABLE 1**

| Gene ID | Species | Gene Name | Expected to be Present | S-N Standard Protocol | S-N Amplified Protocol | Ratio Amp:Std |
|---|---|---|---|---|---|---|
| 1 | Human | MCI-1 | Yes | 2417 | 7000 | 2.90 |
| 2 | Human | p53-induced Protein | Yes | 494 | 1397 | 2.83 |
| 3 | Human | eNOS | Yes | 5656 | 16985 | 3.00 |
| 4 | Human | BCE | Yes | 1137 | 3298 | 2.90 |
| 5 | Human | beta-NGF | Yes | 7694 | 23558 | 3.06 |
| 6 | Human | Telomerase R | Yes | 18290 | 64288 | 3.51 |
| 7 | Human | GMCSF | Yes | 94 | 251 | 2.66 |
| 8 | Human | IGF-1 | Yes | 133 | 301 | 2.26 |
| 9 | HPV | HPV16 E6 mRNA | Yes | 2427 | 8776 | 3.62 |
| 10 | HPV | HPV16-E7 mRNA | Yes | 741 | 2659 | 3.59 |
| 11 | HPV | HPV16 L1 mRNA | Yes | 68 | 299 | 4.38 |
| 12 | HPV | HPV16 L2 mRNA | Yes | 179 | 632 | 3.54 |
| 13 | Human | GADPH | Yes | 23301 | 64938 | 2.79 |
| 14 | B. Subtilis | pGIBS-phe | No | 21 | 20 | NA |
| 15 | B. Subtilis | pGIBS-trp | No | 8 | -62 | NA |
| 16 | B. Subtilis | pGIBS-lys | No | 13 | -50 | NA |
| 17 | Human | beta- actin | Yes | 13252 | 48630 | 3.67 |
| 18 | E. Coli | pGIKS-BioC | No | 10 | -20 | NA |
| 19 | E. Coli | pGIKS-BioD | No | 9 | -41 | NA |
| 20 | E. Coli | pGIKS-BioB | No | -2 | -31 | NA |
| 21 | Human | Ribosomal Protein L37 | Yes | 20197 | 64872 | 3.21 |
| 22 | Human | Ribosomal Protein L 10 | Yes | 6749 | 24427 | 3.62 |
| 23 | A. Thaliana | Xyloglucan endotransglycos ylase-related protein (XTR7) | No | 49 | 45 | NA |
| 24 | A. Thaliana | putative 60S ribosomal protein L1 | No | 11 | -22 | NA |
| 25 | A. Thaliana | GAPDH C subunit (GapC) gene. | No | 16 | -34 | NA |
| 26 | A. Thaliana | eukaryotic translation initiation factor 4A-1 | No | 9 | -54 | NA |
| 27 | A. Thaliana | Alpha- 1-tubulin gene, | No | 8 | -43 | NA |
| 28 | A. Thaliana | Nitrate reductase mRNA | No | 6 | -39 | NA |
| 29 | Human | Ribosomal Protein S11 | Yes | 7529 | 31493 | 4.18 |
| 30 | Human | Phospholipase A 2 | Yes | 4645 | 21516 | 4.63 |

### EXAMPLE 2

### PROTEIN DETECTION WITH HYBRID CAPTURE SIGNAL AMPLIFICATION

In order to prepare the goat serum-coated plates, normal goat serum (Invitrogen) was serially diluted 1:10 in filtered phosphate buffered saline containing 0.05% sodium azide (PBSA) to give final concentrations of 1 (100%), 0.1 (10%), 0.01 (1%), 0.001 (0.1%), 0.0001 (0.01 %), and 0.00001 (0.001%).

Aliquots (100 µl) of the neat goat serum and the goat serum dilutions were added to the bottom of a Coming High Binding white polystyrene microplate (Corning;) such that there were two sets of eight samples for each dilution. The microtiter plates were then incubated overnight at room temperature. The next day, the plates were decanted and 300 µl Post-Coat Solution (DIGENE, Corp.; Gaithersburg, MD) was added to each well. The plates were incubated for 4 hours at room temperature. The plates were again decanted and were then allowed to dry overnight at room temperature before being sealed into Mylar bags with dessicant.

A 100-base sequence of the human c-myc mRNA with a biotin label on the 5' end was prepared by chemical synthesis as well known in the art (oligos, etc.). The biotinylated c-myc oligo was diluted in RNAse- DNAse-free water at a concentration of 420 µg/mL.

An anti-sense strand of c-myc mRNA was prepared from a DNA clone containing a 1290 base sequence coding for human c-myc mRNA. RNA was transcribed from the DNA clone using T7 RNA polymerase and standard RNA transcription techniques. The transcribed RNA was diluted to 420 µg/mL in 3.3X SSC.

Equal volumes of biotin-labeled c-myc oligonucleotide and c-myc RNA were mixed and 100 µl aliquots were placed into 6 PCR tubes. The 6 PCR tubes were placed in a thermal cycler and the hybridization between the RNA and DNA was accomplished using the following incubation profile:

| PCR CYCLING CONDITIONS | |
|---|---|
| 95°C | 5 minutes |
| 75°C | 10 minutes |
| 70°C | 10 minutes |
| 65°C | 10 minutes |
| 60°C | 10 minutes |
| 55°C | 10 minutes |
| 50°C | 10 minutes |
| 45°C | 10 minutes |
| 40°C | 10 minutes |
| 4°C | hold |

Excess RNA was removed by digestion of the reaction mix with Qiagen P1 buffer containing RNAse A for 30 minutes at 37°C. Removal of excess RNA was verified by gel analysis. The RNA:DNA hybrids were precipitated with 3M Sodium Acetate, washed with 70% ethanol, and resuspended in 1X TE buffer. The concentration of RNA:DNA hybrids was measured as 545 µg/mL by spectrophotometric analysis at 260 nm.

The following solutions were prepared. Biotinylated rabbit anti-goat antibody (Vector Laboratories) was diluted to 100 and 10 µg/mL in PBSA. Streptavidin was diluted to 5 µg/mL in PBSA. Biotinylated RNA:DNA Hybrids (c-myc) were diluted to 2 µg/mL in PBSA. Hybrid Capture EAS Detection Reagent 1 (DIGENE, Corp.) containing alkaline-phosphatase conjugated anti-RNA:DNA antibody was used as supplied from the manufacturer.

The assay for Goat IgG bound to the microplate by Hybrid Capture Signal amplification was conducted as follows. Biotin-anti-goat antibody (Vector Laboratories) or PBSA (75 µl) was added to each well The plate was incubated for 30 minutes at room temperature with shaking at 1100 rpm. The plate was then washed 4 times with Wash Buffer (40 mM Tris, 100 mM NaCl, 0.05% Sodium Azide). Streptavidin (Prozyme) or PBSA (75 µl) was added to each well. The plate was incubated for 30 minutes at room temperature with shaking at 1100 rpm. The plate was washed 4 times with Wash Buffer. A mixture of Hybrid Capture EAS Detection Reagent 1 containing alkaline phosphatase anti-RNA:DNA antibody conjugate (DIGENE, Corp.) and biotin-RNA:DNA hybrids or water (75 µl) was added to each well. The plate was incubated for 30 minutes at room temperature with shaking at 1100 rpm. The plate was washed 4 times with Wash Buffer. The plate was washed 6 times with Wash Buffer. CDP-Star with Emerald II Chemiluminescent Substrate (Applied Biosystems; 75 µl) was added to each well. The plate was incubated for 15 minutes at room temperature. Light emission was measured using a DIGENE DML-2000 plate luminometer.

The experiment was laid out as described in Table 2 where Rows A and E contain all elements of the assay. Rows B, C, D, F, G, and H are all controls which are missing at least one element of the assay.

**TABLE 2**

| | | Assay Concentration (µg/mL) | | |
|---|---|---|---|---|
| Row | | Biotin anti-goat- | Streptavidin | RNA:DNA Hybrid |
| A | Exp | 100 | 5 | 2 |
| B | Control | 100 | 0 | 2 |
| C | Control | 0 | 5 | 2 |
| D | Control | 100 | 5 | 0 |
| E | Exp | 10 | 5 | 2 |
| F | Control | 10 | 0 | 2 |
| G | Control | 10 | 5 | 0 |
| H | Control | 0 | 0 | 2 |

Results from duplicate wells were averaged and are represented by mean relative light units in Table 3.

**TABLE 3**

| | **Goat Serum Concentration** | | | | | |
|---|---|---|---|---|---|---|
| | **1** | **0.1** | **0.01** | **0.001** | **0.0001** | **0.00001** |
| **A** | 131280 | 35006 | 291328 | 446141 | 392574 | 79214 |
| **B** | 3406 | 347 | 1212 | 1409 | 1126 | 245 |
| **C** | 3770 | 530 | 1241 | 1649 | 1361 | 657 |
| **D** | 4178 | 400 | 1176 | 1287 | 1111 | 207 |
| **E** | 13041 | 2685 | 19824 | 29521 | 28998 | 7012 |
| **F** | 3148 | 387 | 1021 | 1317 | 1096 | 175 |
| **G** | 2749 | 585 | 988 | 1205 | 963 | 143 |
| **H** | 3252 | 394 | 1037 | 1245 | 1021 | 124 |

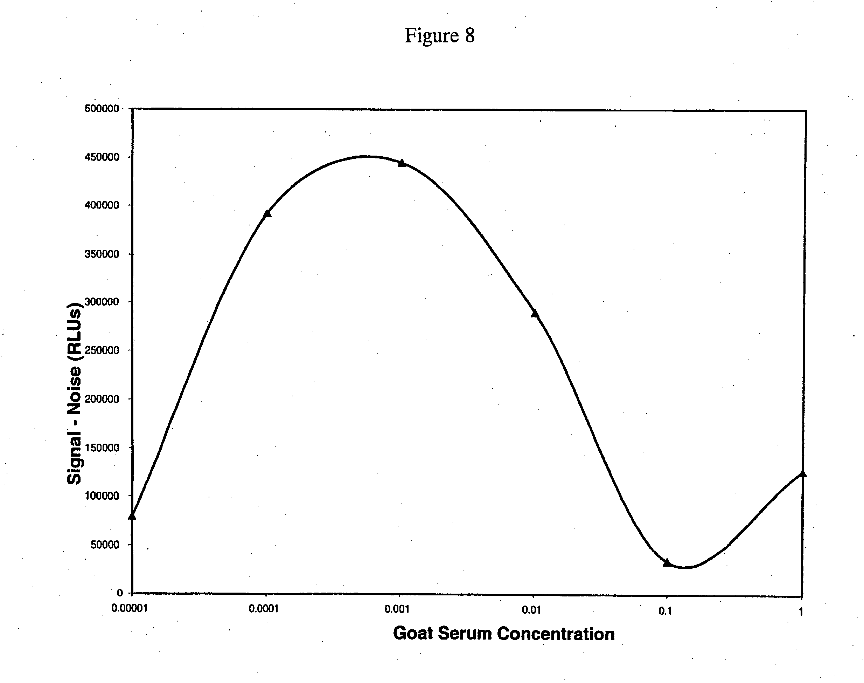

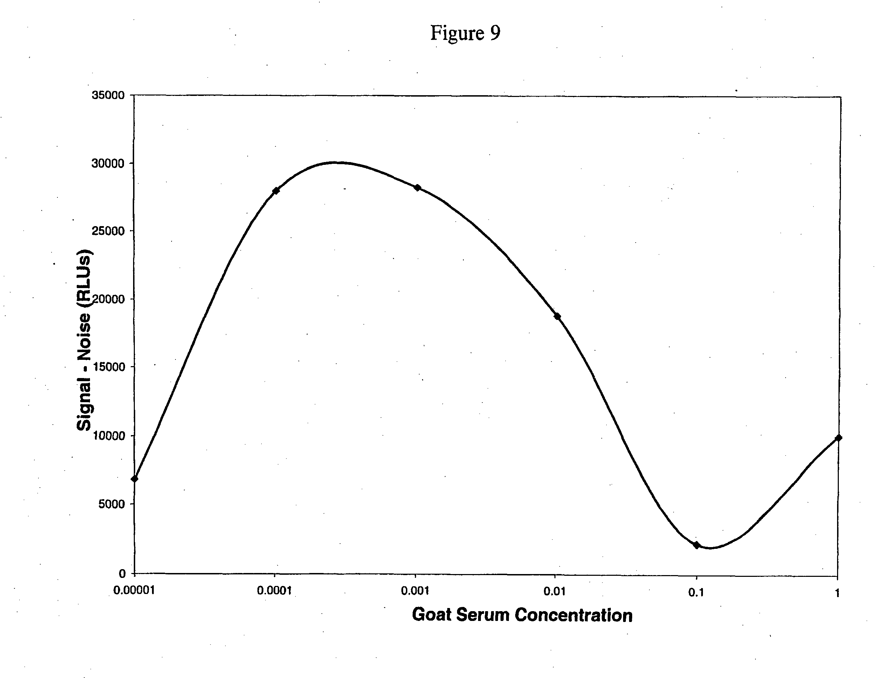

In order to measure net signal, the average of the controls was calculated for each concentration of primary antibody (biotin-anti-goat) and each concentration of bound goat serum (see Tables 4 and 5; Figures 8 and 9). Therefore, for 100 µg/mL biotin-anti-goat, the signals from rows B and D were averaged. For 10 µg/mL biotin-anti-goat, the signals from rows F an G were averaged.

**TABLE 4**

| | **Goat Serum Concentration / 100 µg/mL biotin-anti-goat** | | | | | |
|---|---|---|---|---|---|---|
| | **1** | **0.1** | **0.01** | **0.001** | **0.0001** | **0.00001** |
| **Signal** | 131280 | 35006 | 291328 | 446141 | 392574 | 79214 |
| **Noise** | 3792 | 374 | 1194 | 1348 | 1119 | 226 |
| **S-N** | 127488 | 34633 | 290134 | 444793 | 391456 | 78988 |

**TABLE 5**

| | **Goat Serum Concentration / 10 µg/mL biotin-anti-goat** | | | | | |
|---|---|---|---|---|---|---|
| | **1** | **0.1** | **0.01** | **0.001** | **0.0001** | **0.00001** |
| **Signal** | 13041 | 2685 | 19824 | 29521 | 28998 | 7012 |
| **Noise** | 2949 | 486 | 1005 | 1261 | 1030 | 159 |
| **S-N** | 10093 | 2199 | 18820 | 28260 | 27969 | 6853 |

The results summarized in Tables 3-5 and Figures 8 and 9 demonstrate that an anti-RNA:DNA antibody may be used to detect proteins through hybrid capture signal amplification. At high serum concentrations, the amount of IgG that bound to the solid phase is limited by competition with other serum proteins. As the serum is diluted, a greater proportion of the IgG binds to the solid phase resulting in an increase in signal intensity. At even greater dilution, the signal decreases due to the declining amount of IgG that is available to bind. The controls that lack one or more components of the assay (Tables 2 and 3, rows, B-D, G-H) all gave similar results that were dramatically lower than the experiments that contained all assay components. These results demonstrate that detection was highly specific, since if any component of the assay was left out, only background signals were obtained.

## Claims

1. A method of signal amplification for detecting a target analyte, comprising:
(a) reacting a target analyte with a conjugated binding agent, wherein said binding agent is an antibody which is conjugated to a binding molecule, and wherein said antibody is reactive with the target analyte;
(b) reacting said binding molecule with a first binding partner forming a binding pair between the binding molecule and the first binding partner, wherein the binding partner is conjugated to a reagent, wherein the reagent is a nucleic acid hybrid, which has a repeating motif created by the structure or conformation of the nucleic acid hybrid;
(c) reacting the reagent with a plurality of reagent-specific detectably labeled binding entities forming detectable complexes; and
(d) detecting the plurality of detectable complexes, thereby providing signal amplification.

2. The method according to claim 1, further comprising, reacting a portion of the target analyte with an immobilized antibody, forming an immobilized target-binding agent complex.

3. The method according to claim 1, further comprising:
immobilizing a target nucleic acid by hybridizing the nucleic acid with an oligonucleotide forming a nucleic acid-oligonucleotide hybrid, wherein the oligonucleotide is complementary to a first portion of the target, and is conjugated to a second binding partner, wherein said second binding partner is bound to an immobilized binding molecule forming an immobilized binding pair.

4. The method according to claim 3, wherein the nucleic acid-oligonucleotide hybrid forms the target analyte.

5. The method according to claim 3, further comprising:
hybridizing a nucleic acid probe complementary to a second portion of the target nucleic acid, forming the target analyte, which is then reactive with the conjugated binding agent.

6. The method according to any one of claims 1 to 5, wherein said method is a microarray method.

7. The method according to claim 1, 2, or 6, wherein the target analyte is selected from the group consisting of: lipids, sterols, hormones, drugs, chemicals, toxins, and cells.

8. The method according to claim 1, 2, or 6, wherein the target analyte is selected from the group consisting of: a peptide, a PNA, amino acids, analogues, glycoproteins, proteoglycans, and sugars.

9. The method according to any one of claims 1 to 6, wherein the target analyte is a nucleic acid, said target nucleic acid is selected from the group consisting of: a nucleic acid, a nucleic acid hybrid, a RNA:DNA hybrid, and a DNA:DNA hybrid.

10. The method according to any one of claims 1 to 6, wherein the nucleic acid hybrid is selected from the group consisting of a RNA:DNA hybrid; a RNA: RNA hybrid; a DNA:DNA hybrid; a RNA:DNA vector hybrid; and a M13 RNA:DNA vector hybrid.

11. The method according to any one of claims 1 to 6, wherein the binding entities are antibodies or fragments thereof.

12. The method according to any one of claims 1 to 6, wherein the binding pair is any one selected from the group consisting of: streptavidin:biotin; an avidin:biotin; a folic acid:folate binding protein; a sialic acid, a carbohydrate, or glycoprotein:lectin; an oligo- or poly-dA:oligo- or poly-dT; an oligo- or poly-dC:oligo- or poly-dG; a Phenylboronic acid:Salicylhydroxamic acid; an aldehyde and ketone moiety:hydrazide; a sulfhydryl moiety:maleimide; an amino moiety:N-hydroxysuccinimide esters; and a heavy metal:thiol; an Fc portion of IgG:Protein A/ Protein G/Protein A/G; a digoxigenin:anti-digoxigenin; a 5-bromodeoxyuridine:anti-bromodeoxyuridine; a Dinitrophenyl:anti-Dinitrophenyl; a Fluorescein isothiocyanate:anti-Fluorescein isothiocyanate; an N-2-Acetylaminofluoren:anti-N-2-Acetylaminofluoren; and an N-2-Acetylamino-7-iodofluoren:anti-N-2-Acetylamino-7-iodofluoren.

13. The method according to claim 1, wherein the target analyte is immobilized to a solid phase.

14. The method according to claim 13, wherein the solid phase is any one selected from the group consisting of a microwell or microtiter plates or dishes, silicon chips, glass slides, beads, microparticles, films or membranes, bottles, dishes, slides, blotting material, filters, fibers, woven fibers, shaped polymers, particles, dip- sticks, test tubes, chips, microchips, and a liposome.

15. A composition for performing a signal amplification assay according to claim 1, comprising:
a) a binding agent, wherein said binding agent is an antibody, conjugated to a binding molecule;
b) a conjugated binding partner that reacts with the binding molecule comprising a reagent and a binding partner, wherein the reagent is a nucleic acid hybride which has a repeating motif created by the structure or conformation of the nucleic acid hybride; and
c) a plurality of reagent-specific detectably labeled binding entities, wherein the plurality of reagent-specific detectably labeled binding entities reacts with the reagent forming detectable complexes, said detectable complexes providing signal amplification.

16. The composition according to claim 15, wherein said nucleic acid hybrid is selected from the group consisting of: an RNA:DNA hybrid, a DNA:DNA hybrid; an RNA: RNA hybrid; an RNA:DNA vector hybrid; and a M13 RNA:DNA vector hybrid.

17. The composition according to claim 15, wherein a binding pair comprises the binding partner and a binding molecule, wherein the binding pair is selected from a group consisting of: a streptavidin:biotin; an avidin:biotin; a folic acid:folate binding protein; a sialic acid, a carbohydrate, or glycoprotein:lectin; an oligo- or poly-dA:oligo- or poly-dT; an oligo- or poly-dC:oligo- or poly-dG; a Phenylboronic acid:Salicylhydroxamic acid; an aldehyde and ketone moiety:hydrazide; a sulfhydryl moiety:maleimide; an amino moiety:N-hydroxysuccinimide esters; and a heavy metal:thiol; an Fc portion of IgG:Protein A/ Protein G/Protein A/G; a digoxigenin:anti-digoxigenin; a 5-bromodeoxyuridine:anti-bromodeoxyuridine; a Dinitrophenyl:anti-Dinitrophenyl; a Fluorescein isothiocyanate:anti-Fluorescein isothiocyanate; an N-2-Acetylaminofluoren:anti-N-2-Acetylaminofluoren; and an N-2-Acetylamino-7-iodofluoren:anti-N-2-Acetylamino-7-iodofluaren.

18. The composition according to claim 15, wherein the binding entity is selected from a group consisting of: an RNA:DNA hybrid antibody; Psoralen; ethidium bromide; propidium iodide; Hoechst dyes; SYBR Green; and a cis-platinum derivative.

19. The composition according to claim 15, wherein the detectable label of the plurality of reagent-specific detectably labeled binding entities is selected from a group consisting of: a radioactive isotope; an enzyme; a fluorogenic; a chemiluminescent; an electrochemical material; a micro- or nano-transponder; a nanocrystal; and a fluorescent or light-scatterer.

## Patentansprüche

1. Verfahren zur Signalamplifikation zum Nachweis eines Zielanalyten umfassend:
(a) Reagierenlassen eines Zielanalyten mit einem konjugierten Bindungsagenz, wobei das Bindungsagenz ein Antikörper ist, welcher mit einem Bindungsmolekül konjugiert ist und wobei der Antikörper mit dem Zielanalyten reaktionsfähig ist;
(b) Reagierenlassen des Bindungsmoleküls mit einem ersten Bindungspartner, um ein Bindungspaar zwischen dem Bindungsmolekül und dem ersten Bindungspartner zu bilden, wobei der Bindungspartner mit einem Reagenz konjugiert ist, wobei das Reagenz ein Nukleinsäure-Hybrid ist, welches ein Wiederholungsmotiv aufweist, welches durch die Struktur oder Konformation des Nukleinsäure-Hybrids erzeugt wird;
(c) Reagierenlassen des Reagenz mit einer Vielzahl von Reagenz-spezifischen, detektierbar markierten Bindungseinheiten, die detektierbare Komplexe bilden; und
(d) Detektieren der Vielzahl der detektierbaren Komplexe, wobei Signalamplifikation zur Verfügung gestellt wird.

2. Verfahren nach Anspruch 1, welches ferner das Reagierenlassen eines Teils des Zielanalyten mit einem immobilisierten Antikörper umfasst, um einen immobilisierten Ziel-Bindungsagenz-Komplex zu bilden.

3. Verfahren nach Anspruch 1, ferner umfassend:
Immobilisieren einer Zielnukleinsäure durch Hybridisieren der Nukleinsäure mit einem Oligonukleotid, um ein Nukleinsäure-Oligonukleotid-Hybrid zu bilden, wobei das Oligonukleotid komplementär zu einem ersten Teil des Ziels ist und mit einem zweiten Bindungspartner konjugiert ist, wobei der zweite Bindungspartner an ein immobilisiertes Bindungsmolekül gebunden ist, um ein immobilisiertes Bindungspaar zu bilden.

4. Verfahren nach Anspruch 3, wobei das Nukleinsäure-Oligonukleotid-Hybrid den Zielanalyten bildet.

5. Verfahren nach Anspruch 3, ferner umfassend:
Hybridisieren einer Nukleinsäuresonde, die komplementär zu einem zweiten Teil der Zielnukleinsäure ist, um den Zielanalyten zu bilden, welcher dann mit dem konjugierten Bindungsagenz reaktionsfähig ist.

6. Verfahren nach einem der Ansprüche 1-5, wobei das Verfahren ein Mikroarray-Verfahren ist.

7. Verfahren nach Anspruch 1, 2 oder 6, wobei der Zielanalyt aus der Gruppe bestehend aus: Lipiden, Sterolen, Hormonen, Arzneimitteln, Chemikalien, Toxinen und Zellen ausgewählt ist.

8. Verfahren nach Anspruch 1, 2 oder 6, wobei der Zielanalyt aus der Gruppe bestehend aus: einem Peptid, einer PNA, Aminosäuren, Analoga, Glykoproteinen, Proteoglycanen und Zuckern ausgewählt ist.

9. Verfahren nach einem der Ansprüche 1-6, wobei der Zielanalyt eine Nukleinsäure ist, wobei die Zielnukleinsäure aus der Gruppe bestehend aus: einer Nukleinsäure, einem Nukleinsäure-Hybrid, einem RNA:DNA-Hybrid und einem DNA:DNA-Hybrid ausgewählt ist.

10. Verfahren nach einem der Ansprüche 1-6, wobei das Nukleinsäure-Hybrid aus der Gruppe bestehend aus: einem RNA:DNA-Hybrid, einem RNA:RNA-Hybrid, einem DNA:DNA-Hybrid, einem RNA:DNA-Vektor-Hybrid und einem M 13-RNA:DNA-Vektor-Hybrid ausgewählt ist.

11. Verfahren nach einem der Ansprüche 1-6, wobei die Bindungseinheiten Antikörper oder Fragmente davon sind.

12. Verfahren nach einem der Ansprüche 1-6, wobei das Bindungspaar irgendeines aus der Gruppe bestehend aus: Streptavidin:Biotin, einem Avidin:Biotin, einem Folsäure:Folat-Bindungsprotein, einer Sialinsäure, einem Kohlenhydrat oder Glycoprotein:Lectin, einem Oligo- oder PolydA:Oligo- oder Poly-dT, einem Oligo- oder Poly-dC:Oligo- oder Poly-dG, einer Phenylboronsäure:Salicylhydroxamsäure, einem Aldehyd- und Keton-Rest:Hydrazid, einem Sulfhydryl-Rest:Maleimid, einem Amino-Rest:N-Hydroxysuccinimidester und einem Schwermetall:Thiol, einem Fc-Teil des IgG:Protein A/Protein G/Protein A/G, einem Digoxigenin:Anti-Digoxigenin, einem 5-Bromdeoxyuridin:Anti-Bromdeoxyuridin, einem Dinitrophenyl:Anti-Dinitrophenyl, einem Fluoresceinisothiocyanat:Anti-Fluoresceinisothiocyanat, einem N-2-Acetylaminofluoren:Anti-N-2-Acetylaminofluoren und einem N-2-Acetylamino-7-iodfluoren:Anti-N-2-Acetylamino-7-iodfluoren ist.

13. Verfahren gemäß Anspruch 1, wobei der Zielanalyt an einer festen Phase immobilisiert ist.

14. Verfahren gemäß Anspruch 13, wobei die feste Phase irgendeine ausgewählt aus der Gruppe bestehend aus: Microwell- oder Microtiter-Platten oder Teller, Silikonchips, Glasträger, Beads, Mikropartikel, Filme oder Membranen, Flaschen, Teller, Träger, Blotting-Material, Filter, Fasern, gewebte Fasern, geformte Polymere, Partikel, Messstäbchen, Teströhrchen, Chips, Mikrochips und einem Liposom ist.

15. Zusammensetzung zur Durchführung eines Verfahrens zur Signalamplifikation gemäß Anspruch 1, umfassend:
a) ein Bindungsagenz, wobei das Bindungsagenz ein Antikörper ist, der mit einem Bindungsmolekül konjugiert ist;
b) einen konjugierten Bindungspartner, der mit dem Bindungsmolekül reagiert, welches ein Reagenz und einen Bindungspartner umfasst, wobei das Reagenz ein Nukleinsäure-Hybrid ist, welches ein Wiederholungsmotiv aufweist, welches durch die Struktur oder Konformation des Nukleinsäure-Hybrids erzeugt wird; und
c) eine Vielzahl von Reagenz-spezifischen, detektierbar markierten Bindungseinheiten, wobei die Vielzahl der Reagenz-spezifischen, detektierbar markierten Bindungseinheiten mit dem Reagenz reagiert, um detektierbare Komplexe zu bilden, wobei die detektierbaren Komplexe Signalamplifikation zur Verfügung stellen.

16. Zusammensetzung gemäß Anspruch 15, wobei das Nukleinsäure-Hybrid aus der Gruppe bestehend aus: einem RNA:DNA-Hybrid, einem DNA:DNA-Hybrid, einem RNA:RNA-Hybrid, einem RNA:DNA-Vektor-Hybrid und einem M 13-RNA:DNA-Vektor-Hybrid ausgewählt ist.

17. Zusammensetzung gemäß Anspruch 15, wobei ein Bindungspaar den Bindungspartner und ein Bindungsmolekül umfasst, wobei das Bindungspaar aus einer Gruppe bestehend aus: einem Streptavidin:Biotin, einem Avidin:Biotin, einem Folsäure:Folat-Bindungsprotein, einer Sialinsäure, einem Kohlenhydrat oder Glycoprotein:Lectin, einem Oligo- oder Poly-dA:Oligo- oder Poly-dT, einem Oligo- oder Poly-dC:Oligo- oder Poly-dG, einer Phenylboronsäure:Salicylhydroxamsäure, einem Aldehyd- und Keton-Rest:Hydrazid, einem Sulfhydryl-Rest:Maleimid, einem Amino-Rest:N-Hydroxysuccinimidester und einem Schwermetall:Thiol, einem Fc-Teil eines IgG:Protein A/Protein G/Protein A/G, einem Digoxigenin:Anti-Digoxigenin, einem 5-Bromdeoxyuridin:Anti-Bromdeoxyuridin, einem Dinitrophenyl:Anti-Dinitrophenyl, einem Fluoresceinisothiocyanat:Anti-Fluoresceinisothiocyanat, einem N-2-Acetylaminofluoren:Anti-N-2-Acetylaminofluoren und einem N-2-Acetylamino-7-iodfluoren:Anti-N-2-Acetylamino-7-iodfluoren ausgewählt ist.

18. Zusammensetzung gemäß Anspruch 15, wobei die Bindungseinheit aus einer Gruppe bestehend aus: einem RNA:DNA-Hybrid-Antikörper, Psoralen, Ethidiumbromid, Propidiumiodid, Hoechst-Farbstoffe, SYBR-Green und einem Cis-Platin-Derivat ausgewählt ist.

19. Zusammensetzung gemäß Anspruch 15, wobei die detektierbare Markierung der Vielzahl an Reagenz-spezifischen, detektierbar markierten Bindungseinheiten aus einer Gruppe bestehend aus: einem radioaktiven Isotop, einem Enzym, einem Fluorogen, einem Chemilumineszenten, einem elektrochemischen Material, einem Mikro- oder Nano-Transponder, einem Nanokristall und einem Fluoreszenz- oder Licht-Zerstreuer ausgewählt ist.

## Revendications

1. Procédé d'amplification de signal pour la détection d'un analyte cible, comprenant:
(a) la réaction d'un analyte cible avec un agent de liaison conjugué, tandis que ledit agent de liaison est un anticorps qui est conjugué à une molécule de liaison, et tandis que ledit anticorps est réactif avec l'analyte cible;
(b) la réaction de ladite molécule de liaison avec un premier partenaire de liaison formant une paire de liaison entre la molécule de liaison et le premier partenaire de liaison, tandis que le partenaire de liaison est conjugué à un réactif, le réactif étant un hybride d'acide nucléique, qui a un motif répété créé par la structure ou la conformation de l'hybride d'acide nucléique;
(c) la réaction du réactif avec une pluralité d'entités de liaison marquées de manière détectable et spécifiques pour le réactif, et formant des complexes détectables; et
(d) la détection de la pluralité de complexes détectables, procurant ainsi une amplification du signal.

2. Procédé selon la revendication 1, comprenant en outre la réaction d'une portion de l'analyte cible avec un anticorps immobilisé, formant ainsi un complexe immobilisé d'agent de liaison-cible.

3. Procédé selon la revendication 1, comprenant en outre:
l'immobilisation d'un acide nucléique cible par hybridation de l'acide nucléique avec un oligonucléotide pour former un hybride d'acide nucléique-oligonucléotide, tandis que l'oligonucléotide est complémentaire d'une première portion de la cible, et est conjugué à un deuxième partenaire de liaison, tandis que ledit deuxième partenaire de liaison est lié à une molécule de liaison immobilisée pour former une paire de liaison immobilisée.

4. Procédé selon la revendication 3, dans lequel l'hybride d'acide nucléique-oligonucléotide forme l'analyte cible.

5. Procédé selon la revendication 3, comprenant en outre:
l'hybridation d'une sonde d'acide nucléique complémentaire d'une deuxième portion de l'acide nucléique cible, pour former l'analyte cible, qui est alors réactif avec l'agent de liaison conjugué.

6. Procédé selon l'une quelconque des revendications 1 à 5, dans lequel ledit procédé est un procédé avec microarrangement.

7. Procédé selon la revendication 1, 2 ou 6, dans lequel l'analyte cible est choisi dans le groupe consistant en: lipides, stérols, hormones, médicaments, produits chimiques, toxines, et cellules.

8. Procédé selon la revendication 1, 2 ou 6, dans lequel l'analyte cible est choisi dans le groupe consistant en: un peptide, un PNA, des aminoacides, des analogues, des glycoprotéines, des protéoglycanes, et des sucres.

9. Procédé selon l'une quelconque des revendications 1 à 6, dans lequel l'analyte cible est un acide nucléique, ledit acide nucléique cible étant choisi dans le groupe consistant en: un acide nucléique, un hybride d'acide nucléique, un hybride d'ARN:ADN, et un hybride d'ADN:ADN.

10. Procédé selon l'une quelconque des revendications 1 à 6, dans lequel l'hybride d'acide nucléique est choisi dans le groupe consistant en un hybride d'ARN:ADN, un hybride d'ARN:ARN, un hybride d'ADN:ADN, un hybride de vecteur d'ADN:ARN, et un hybride de vecteur d'ADN:ARN de M13.

11. Procédé selon l'une quelconque des revendications 1 à 6, dans lequel les entités de liaison sont des anticorps ou des fragments de ceux-ci.

12. Procédé selon l'une quelconque des revendication 1 à 6, dans lequel la paire de liaison est l'une quelconque choisie dans le groupe consistant en: streptavidine:biotine; une avidine:biotine; un acide folique:protéine de liaison de folate; un acide sialique, un hydrate de carbone, ou une glycoprotéine:lectine; une oligo- ou poly-dA:oligo- ou poly-dT; une oligo- ou poly-dC:oligo- ou poly-dG; un acide phénylboronique:un acide salicylhydroxamique; une entité aldéhyde et cétone:hydrazide; une entité sulfhydryle:maléimide; une entité amino:esters de N-hydroxysuccinimide; et un métal lourd:thiol; une portion Fc d'IgG:protéine A/ protéine G/ protéine A/G; une digoxigénine:anti-digoxigénine; une 5-bromodésoxyuridine:anti-bromodésoxyuridine; un dinitrophényle:anti-dinitrophényle; un isothiocyanate de fluorescéine:anti-isothiocyanate de fluorescéine; un N-2-acétylaminofluorène:N-2-acétylaminofluorène; et un N-2-acétylamino-7-iodofluorène:N-2-acétylamino-7-iodofluorène.

13. Procédé selon la revendication 1, dans lequel l'analyte cible est immobilisé sur une phase solide.

14. Procédé selon la revendication 13, dans lequel la phase solide est l'une quelconque choisie dans le groupe consistant en un micropuits ou des plaques ou boîtes de microtitrage, des puces de silicium, des lames de verre, des billes, des microparticules, des films ou des membranes, des flacons, des boîtes, des lames, du matériel pour buvardage, des filtres, des fibres, des fibres tissées, des polymères conformés, des particules, des bâtons à plonger, des tubes à essai, des puces, des micropuces, et un liposome.

15. Composition pour la réalisation d'un essai d'amplification de signal selon la revendication 1, comprenant:
a) un agent de liaison, ledit agent de liaison étant un anticorps, conjugué à une molécule de liaison;
b) un partenaire de liaison conjugué qui réagit avec la molécule de liaison, comprenant un réactif et un partenaire de liaison, le réactif étant un hybride d'acide nucléique qui a un motif répété créé par la structure ou la conformation de l'hybride d'acide nucléique; et
c) une pluralité d'entités de liaison marquées de manière détectable et spécifiques pour un réactif, tandis que la pluralité d'entités marquées de manière détectable et spécifiques pour un réactif réagit avec le réactif pour former des complexes détectables, lesdits complexes détectables procurant une amplification de signal.

16. Composition selon la revendication 15, dans laquelle ledit hybride d'acide nucléique est choisi dans le groupe consistant en: un hybride d'ARN:ADN, un hybride d'ADN:ADN, un hybride d'ARN:ARN, un hybride de vecteur d'ADN:ARN, et un hybride de vecteur d'ADN:ARN de M13.

17. Composition selon la revendication 15, dans laquelle une paire de liaison comprend le partenaire de liaison et une molécule de liaison, tandis que la paire de liaison est choisie dans un groupe consistant en:
streptavidine:biotine; une avidine:biotine; un acide folique:protéine de liaison de folate; un acide sialique, un hydrate de carbone, ou une glycoprotéine:lectine; une oligo- ou poly-dA:oligo- ou poly-dT; une oligo- ou poly-dC:oligo- ou poly-dG; un acide phénylboronique:salicylhydroxamique; une entité aldéhyde et cétone:hydrazide; une entité sulfhydryle:maléimide; une entité amino:esters de N-hydroxysuccinimide; et un métal lourd:thiol; une portion Fc d'IgG:protéine A/ protéine G/ protéine A/G; une digoxigénine:anti-digoxigénine; une 5-bromodésoxyuridine:anti-bromodésoxyuridine; un dinitrophényle:anti-dinitrophényle; un isothiocyanate de fluorescéine:anti-isothiocyanate de fluorescéine; un N-2-acétylaminofluorène:anti-N-2-acétylaminofluorène; et un N-2-acétylamino-7-iodofluorène:anti-N-2-acétylamino-7-iodofluorène.

18. Composition selon la revendication 15, dans laquelle l'entité de liaison est choisie dans un groupe consistant en: anticorps d'hybride d'ARN:ADN; psoralène; bromure d'éthidium; iodure de propidium; colorants Hoechst; vert SYBR; et un dérivé de cis-platine.

19. Composition selon la revendication 15, dans laquelle le marqueur détectable de la pluralité d'entités de liaison marquées de manière détectable et spécifiques pour un réactif est choisi dans un groupe consistant en: un isotope radioactif; une enzyme; un agent fluorigène; un agent chimioluminescent; un matériel électrochimique; un micro- ou nano-répondeur; un nanocristal; et un agent fluorescent ou dispersant la lumière.
